(19) Europäisches Patentamt
European Patent Office
Office européen des brevets

(11) EP 3 770 165 B1

(12) **EUROPEAN PATENT SPECIFICATION**

(45) Date of publication and mention of the grant of the patent:
**08.06.2022 Bulletin 2022/23**

(21) Application number: **18933805.6**

(22) Date of filing: **25.12.2018**

(51) International Patent Classification (IPC):
***C07F 9/6558*** *(2006.01)* ***A61P 31/10*** *(2006.01)*

(52) Cooperative Patent Classification (CPC):
**C07F 9/65586; A61P 31/10**

(86) International application number:
**PCT/CN2018/123343**

(87) International publication number:
**WO 2020/056974 (26.03.2020 Gazette 2020/13)**

(54) **POSACONAZOLE PHOSPHATE ESTER MONO CHOLINE SALT, PREPARATION METHOD THEREFOR AND USE THEREOF**

POSACONAZOLPHOSPHATEESTERMONOCHOLINSALZ, VERFAHREN ZU SEINER HERSTELLUNG UND SEINE VERWENDUNG

SEL DE MONOCHOLINE D'ESTER DE PHOSPHATE DE POSACONAZOLE, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2018 CN 201811107484**

(43) Date of publication of application:
**27.01.2021 Bulletin 2021/04**

(73) Proprietor: **HC Synthetic Pharmaceutical Co. Ltd.**
**Weinan, Shaanxi 714000 (CN)**

(72) Inventors:
- **YANG, Cheng**
  **Weinan, Shaanxi 714000 (CN)**
- **LU, Hualong**
  **Weinan, Shaanxi 714000 (CN)**

(74) Representative: **Patentwerk B.V.**
**P.O. Box 1514**
**5200 BN 's-Hertogenbosch (NL)**

(56) References cited:
**WO-A1-2017/133632**
**CN-A- 1 161 038**
**CN-A- 101 213 193**
**CN-A- 105 287 403**
**CN-A- 106 432 338**
**CN-A- 107 033 186**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 770 165 B1

## Description

### Field of the Invention

The present invention relates to posaconazole phosphate monocholinate, a preparation method and use thereof.

### Background of the Invention

Fungal infection is a clinically common and frequently-occurring disease. Infection may be divided into two categories: superficial fungal infection and deep tissue fungal infection. The superficial fungal infection is caused by invasion of ringworm fungi to skin, hair, fingernails and other body surfaces, which is of high incidence and minor hazard. The deep tissue fungal infection is caused by invasion of fungi such as candida, aspergillus and cryptococcus to visceral organs and deep tissues, which has major hazards.

In recent years, with the increase in the number of immunocompromised hosts, the incidence rate of deep tissue fungal infection increases dramaticlly. Fungal infection, particularly the deep tissue fungal infection, has attracted extensive attention from day to day. However, at present, clinically used antifungal drugs have problems of significant side effects and susceptibility to drug resistance and the like. Clinically, the existing antifungal drugs, depending on the structures, may be divided into organic acids, polyenes, azoles, allyl amines and the like. The azole antifungal drugs are a class of fully synthetic antifungal compounds that have been developed rapidly and have become major clinical drugs for treating the deep tissue infection and superficial fungal infection at present. Since the antifungal effect of the first azole compound was reported in the middle of the last century, the first-generation triazole drugs such as fluconazole and itraconazole and second-generation triazole drugs such as voriconazole have gradually emerged in the field of antifungal treatment.

Posaconazole is a derivative of itraconazole. Oral suspension of the posaconazole, which first came into the market in Germany in 2005 and was approved for marketing by FDA in 2006, had excellent therapeutic effects on systemic fungal infections caused by the aspergillus and candida and on oropharyngeal candidiasis infection. It was approved in more than 70 countries or regions worldwide and was marked in more than 40 countries or regions, such as America and European Union. However, absorption of the oral suspension is easily affected by factors such as food and gastrointestinal function, thereby causing problems including great difference in pharmacokinetic parameters among individuals, wide-range fluctuation in blood concentration and low bioavailability. Moreover, posaconazole is a drug having weak alkalinity and poor water solubility, and is difficult to be developed as a dosage form of injection, while some immunocompromised hosts receiving chemotherapy or organ transplantation have problems of such as nausea and vomiting and gastrointestinal discomfort that lead to difficulty in oral administration and need to be administered via the route of injection instead.

In order to solve the problem with the difficulty of posaconazole in being developed as a dosage form of injection due to the poor solubility, the patent application CN201180031488.9 filed by MSD Company discloses a posaconazole intravenous infusion formulation solubilized by substituted β-cyclodextrin. In that patent, posaconazole is solubilized by using a substituted β-cyclodextrin, and an injection formulation is prepared. At present, the injection has been approved to be marketed in the United States of America. Although the injection overcomes the defect of insolublility of posaconazole in water and allows administraion to the patients with oral administration inconvenience, the addition of large amount of sulfobutyl ether-β-cyclodextrin (SBE-β-CD) for purpose of solubilization underlies the risk of safety potential. Moreover, preclinical toxicology study shows that the sulfobutyl ether-β-cyclodextrin causes vacuolization of the urothelial cells and activation of the macrophages in the liver and lung. Clinical researches show that the sulfobutyl ether-β-cyclodextrin (SBE-β-CD) needs to be metabolized via kidney, thus the burden of the kidney is greatly increased. However, patients for target indication of the posaconazole injection are immunocompromised hosts who have received bone marrow transplantation, chemotherapy and etc. and have high risk of fungal infection. A substantial portion of the patients have renal function impairments. Particularly, patients suffering from moderate or severe renal insufficiency have lower glomerular filtration efficiency and substantial accumulation of the SBE-β-CD in vivo, underlying higher risk in safety. The use of the excipient of sulfobutyl ether-β-cyclodextrin has greatly limit the range of clinical application of the drug. The directions for use of posaconazole injection particularly points out that this drug is not indicated for patients suffering from moderate or severe renal insufficiency. Therefore, overcoming the defects existing in the prior art and increasing the safety level of drug adminstration and the drug applicability in the renal insufficiency patients are of important clinical value. Posaconazole derivatives are disclosed in CN106432338 A.

### Summary of the Invention

In order to overcome the defects in the prior art, the present invention provides a compound of formula (I) having an effect of resisting fungal infection, a preparation method of the compound, a pharmaceutical composition

including the compound, and the use of the compound in preparation of drugs resistant to fungal infection.

The above objective of the present invention is achievd by the following technical proposals. In one aspect, the present invention provides a compound (posaconazole phosphate monocholinate) of formula (I):

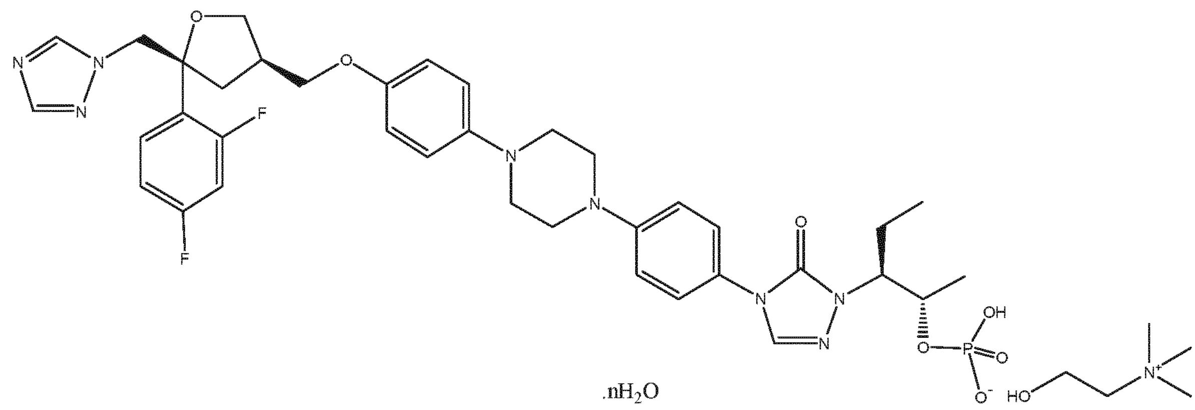

$.nH_2O$

wherein n is an integer of 0~12, preferably an integer of 0~8, more preferably an integer of 0~6; for example, n may be 0, 1, 2, 3, 4, 5, or 6.

In another aspect, the present invention provides a preparation method of the compound of formula (I). The preparation method comprises the following steps:

(a) reacting a compound of formula A with a compound of formula B in the absence of a solvent or in an organic solvent A in the present of an inert gas so as to form a compound of formula C;

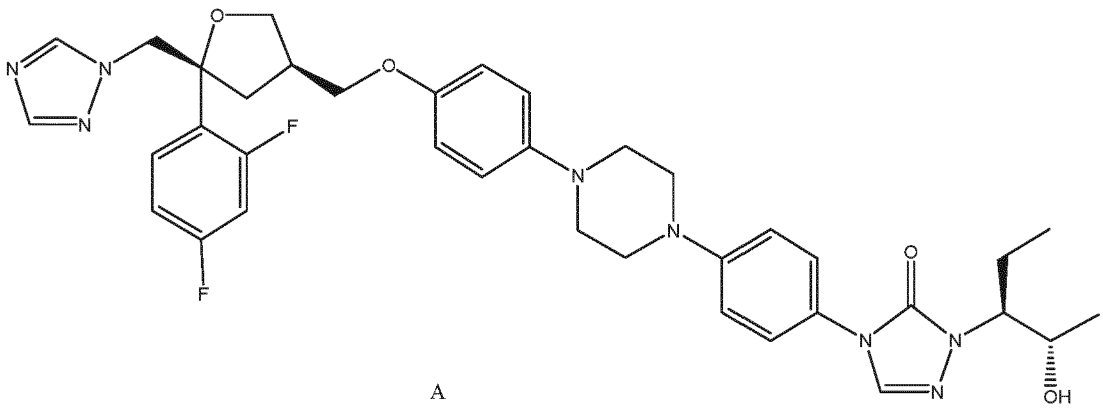

A

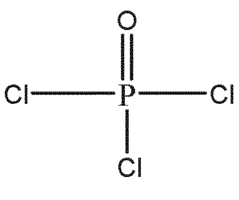

B

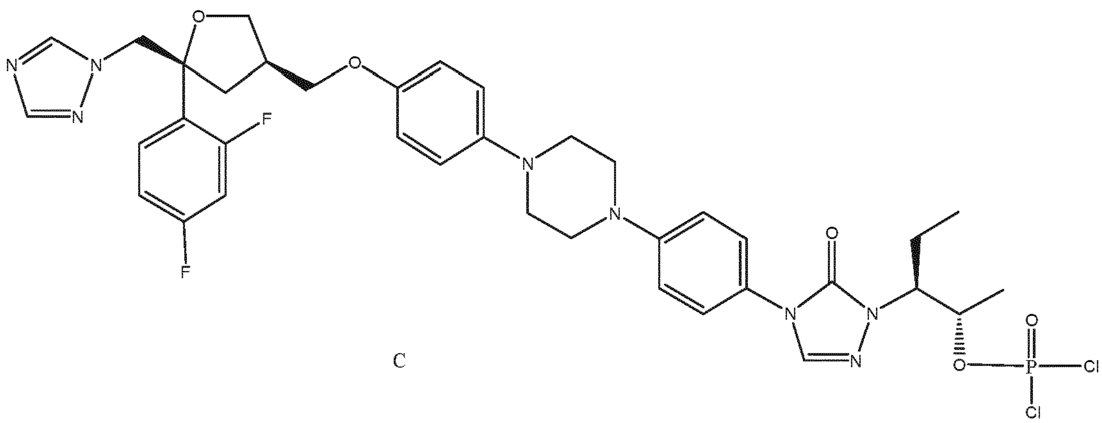

C

(b) hydrolyzing the compound of formula C formed in the step (a) by using a solvent B so as to form a compound of formula D:

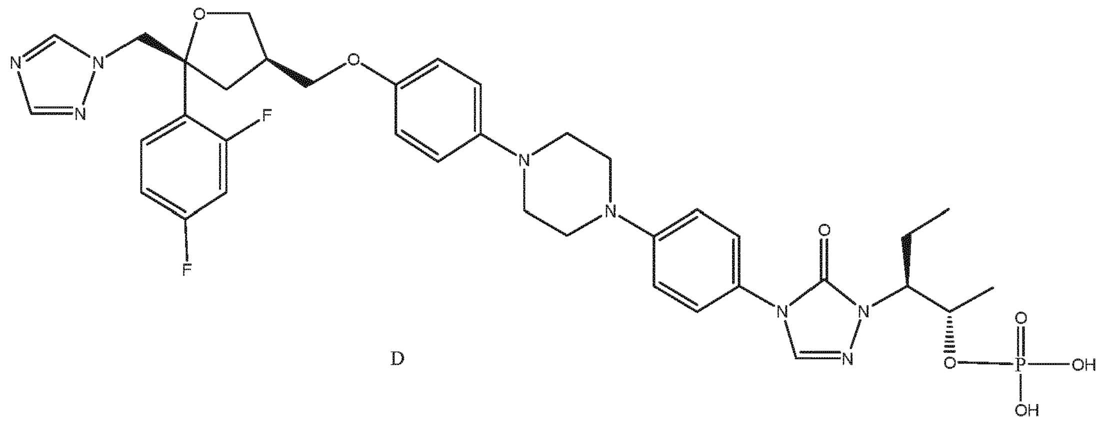

D

(c) reacting the compound of formula D obtained in the step (b) with choline hydroxide in a solvent C so as to prepare the compound of formula (I).

Preferably, in the step (a) of the above preparation method, the inert gas is selected from one or more of nitrogen, helium or argon, preferably nitrogen or argon.

Preferably, in the step (a) of the above preparation method, the organic solvent A is selected from one or more of an aromatic hydrocarbon solvent, a halohydrocarbon solvent, a nitrile solvent, a ketone solvent, an ether solvent, triethylamine, diethylamine, pyridine, 1-methylimidazole, N,N-diisopropyl ethylamine and an ester solvent, preferably ethyl acetate, acetonitrile, tetrahydrofuran, dichloromethane, toluene, acetone, triethylamine, 1-methylimidazole, pyridine or chloroform.

Preferably, in the step (b) of the above preparation method, the solvent B is selected from one or more of water, an alkaline aqueous solution and an aqueous organic solvent solution. The alkaline aqueous solution is preferably an aqueous solution of sodium hydroxide, ammonium hydroxide, an aqueous solution of potassium hydroxide or an aqueous solution of sodium carbonate; and the aqueous organic solvent solution is preferably an aqueous solution of methanol, an aqueous solution of ethanol, an aqueous solution of isopropanol or an aqueous solution of acetone.

Preferably, in the step (c) of the above preparation method, the solvent C is selected from one or more of water, aromatic hydrocarbons, halohydrocarbons, nitriles, ketones, ethers, alcohols and esters; preferably selected from one or more of water, acetonitrile, methanol, ethanol, acetone, isopropanol, tetrahydrofuran, ethyl acetate, dichloromethane, toluene and butanone.

Preferably, in the step (a) of the above preparation method, reaction temperature is -10°C~50°C, preferably 0°C~35°C.

Preferably, in the step (b) of the above preparation method, hydrolysis temperature is -20°C~30°C, preferably -5°C~10°C.

Preferably, in the step (c) of the above preparation method, reaction temperature is -10°C~80°C, preferably 10°C~40°C.

Preferably, in the step (a) of the above preparation method, molar ratio of the compound of formula A to the compound of formula B is 1: (1.0~20.0), preferably 1:(2.25~10.0). Preferably, in the above preparation method, in the step (c), molar ratio of the compound of formula D to the choline hydroxide is 1: (0.5~2), preferably 1:1.05.

According to another aspect, the present invention provides the use of the compound of formula (I) in preparation of drugs resisting fungal infection; preferably, the fungal infection is infection caused by *Candida* or *Cryptococcus.*

According to another aspect, the present invention provides a pharmaceutical composition. The pharmaceutical composition comprises the compound of formula (I) and a pharmaceutically acceptable excipient.

Preferably, the pharmaceutical composition is a tablet, a suppository agent, a dispersible tablet, an enteric-coated tablet, a chewable tablet, an orally disintegrating tablet, a capsule, a sugar-coated tablet, a granule, dry powder, oral solution, a small volume injection, lyophilized powder for injection or a large volume parenteral solution.

Preferably, the pharmaceutically acceptable excipient is selected from one or more of the following excipients: a pH regulator, a diluent, a solubilizer, a disintegrating agent, a suspending agent, a lubricating agent, a binder, a filler, a flavoring agent, a sweetening agent, an antioxidant, a preservative, a coating agent and coloring agent.

The dose and the therapeutic use of the compound of the present invention depend on many factors, including age, body weight, gender, health condition and nutritional status of patients, activity intensity of the compound, duration of use and metabolism rate, severity of the disease, and subjective judgment from a doctor. Preferably, the dose is 2~1200 mg/kg; the preferred dose within 24 hours is 0.2~300 mg per kilogram; or a multiple dosing mode may be adopted.

Compared with the prior arts, the present invention has at least the following beneficial effects.

Firstly, the compound of formula (I) of the present invention, that is, the monocholinate compound, has high solubility and has excellent stability underaffecting factors (such as high temperature, high humidity and illumination) and a long-term storage condition, and the stability is significantly higher than that of among the other of posaconazole monophosphate and the posaconazole di-phosphate.

Secondly, when the monocholinate compound of formula (I) of the present invention is administered into the body, the monocholinate compound functions as a "prodrug", and is converted into biologically active parent posaconazole in the presence of alkaline phosphatase. The monocholinate compound of formula (I) of the present invention has low hydroscopicity, and it can achieve unexpected improved physical stability. Thus, the monocholinate compound is easily treated during the preparation process and maintains suitable solubility, making the prodrug suitable for oral, local and parenteral administration.

## Detailed Description of the Invention

The present invention is further described in detail below in combination with specific examples. The given examples are merely for describing the present invention but not for limiting the scope of the present invention.

**Example 1:** Preparation of posaconazole phosphate monocholinate

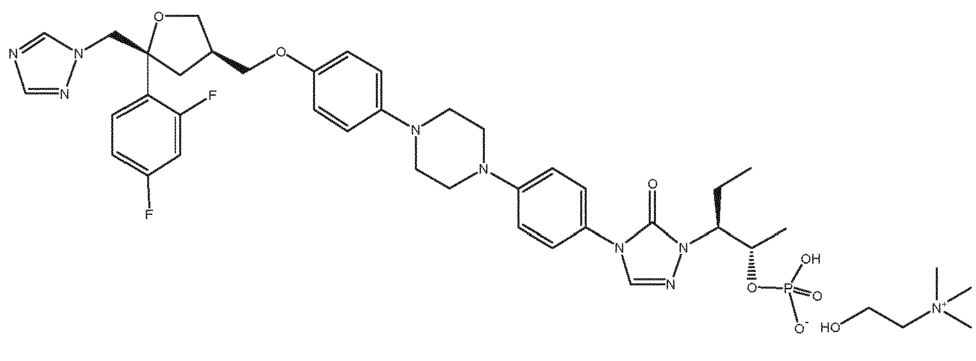

Step 1:

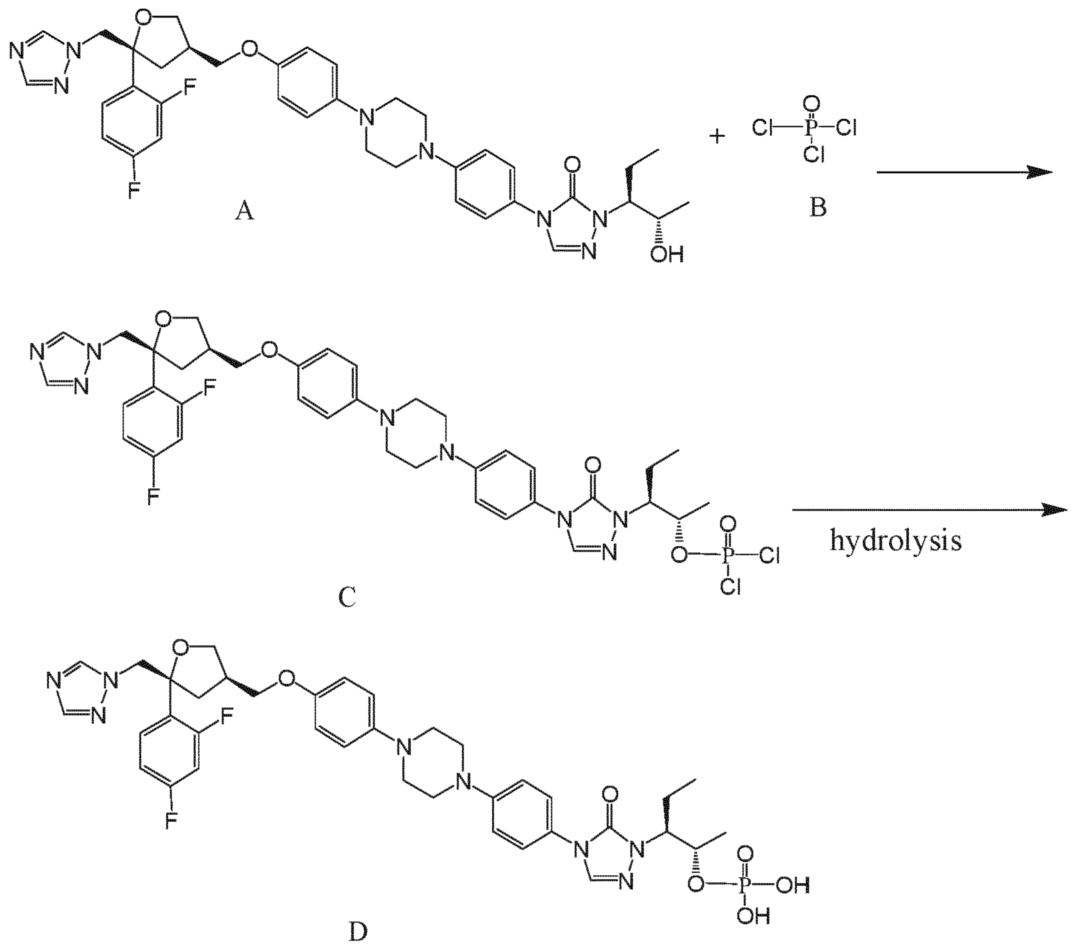


Posaconazole (10g, 14.28 mmol) was weighed and added into a dried 250 ml three-necked flask, and dichloromethane (100 ml) was added under nitrogen protection, and stirring was followed to dissolve. Triethylamine (5 ml) was added at room temperature (25°C), stirring was performed and a reaction was carried out at the room temperature (25°C) for 30 minutes. Then, phosphorus oxychloride (3 ml, 32.13 mmol) was added slowly and the addition was completed within about 1 minute. The resulting mixture was allowed to reac for 6 hours, and the reaction was ended. The progress of the reaction was judged by in-process HPLC.

Chromatographic conditions are as follows:

Mobile phase: 6.8 g/L monopotassium phosphate whose pH value is adjusted with phosphoric acid to 3.0; ratio of monopotassium phosphate to acetonitrile is 60:40.
Detection wavelength: 220 nm; flow velocity: 1.0 ml/min; column temperature: 25°C Sample concentration: 1 mg/ml, dilution medium: 50% of acetonitrile

A reaction solution was dripped into 150 ml of purified water at 0°C, and the hydrolysis temperature was controlled at 0~5°C. The resulting mixture was stirred for 16 hours. An organic phase and an aqueous phase were transferred into a separation funnel for extraction. The solid phase was dissolved with MeOH (150 ml) and combined with the extracted organic phase. About 25 g of 200~300 mesh silica gel was poured into the combined phases. The solvent was evaporated to dryness by using a rotary evaporator. The dried silica gel was poured into a column (having a diameter of 4.5 cm) filled with 25cm thick silica gel pad. When the target product was eluted from the column, rotary evaporation was performed to remove the collected solvent so as to yield a yellow solid to which 150 ml of water and 50 ml of dichloromethane were added. Extraction was conducted, and the organic phase was dried over anhydrous $Na_2SO_4$. The dried organic phase was filtered and filtrate was evaporated to dryness by using a rotary evaporator to yield 6.3g of light yellow solid. Related substances: 0.42% of individual impurity, and 1.02% of total impurities.

Step 2:

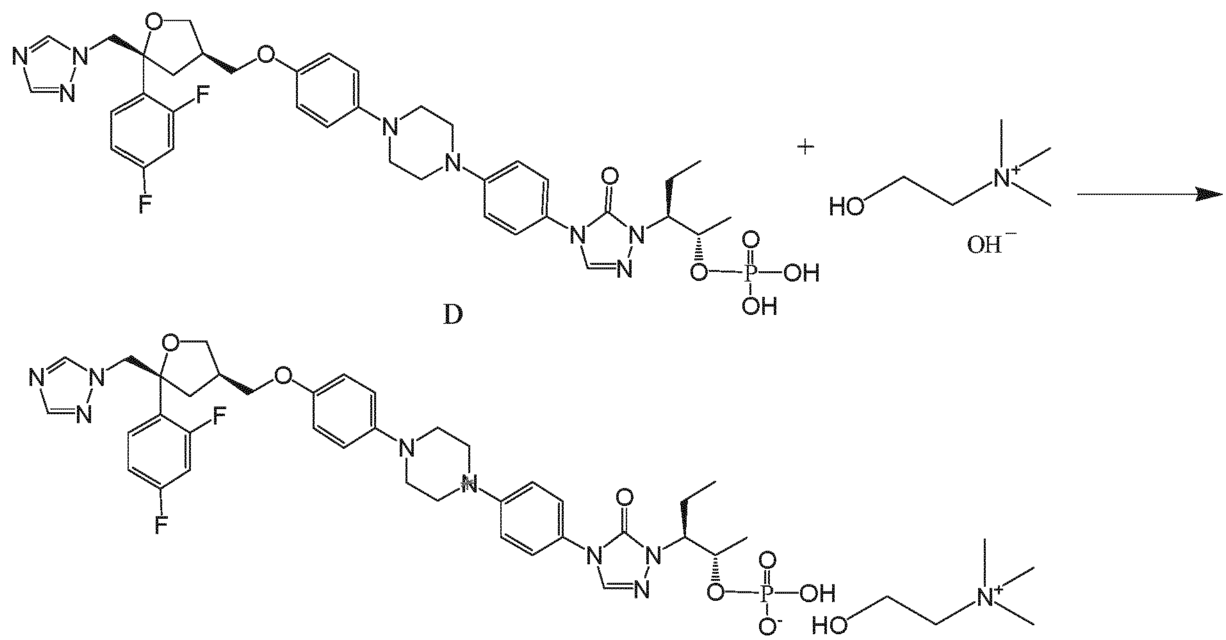

D

Choline hydroxide (0.31 g, 1.28 mmol, 50%) was weighed and added into a beaker filled with 5 ml of purified water, the resulting mixture was stirred uniformly. Compound (formula D) (1 g, 1.28 mmol) was poured into the beaker and stirring was performed at 30°C for 1 hour. After the solid was dissolved, filtration was performed, and the filtrate was poured into a beaker filled with 50 ml of ethanol. The resulting solution was stirrered at room temperature for 12 hours. Suction filtration was performed to yield a white solid which was rinsed with 5 ml of ethanol. The filter cake was dried under vacuum to give 0.76g of white solid (related substances: 0.13%; moisture: 0.3%; content: 99.87%).

**Example 2:** Preparation of posaconazole phosphate monocholinate

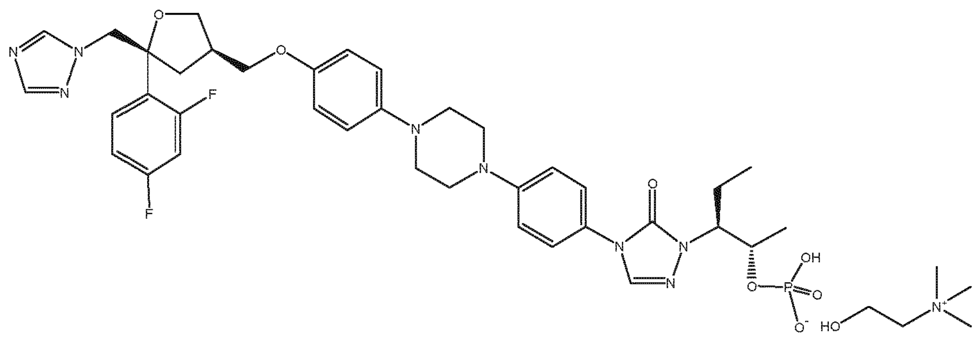

Step 1:

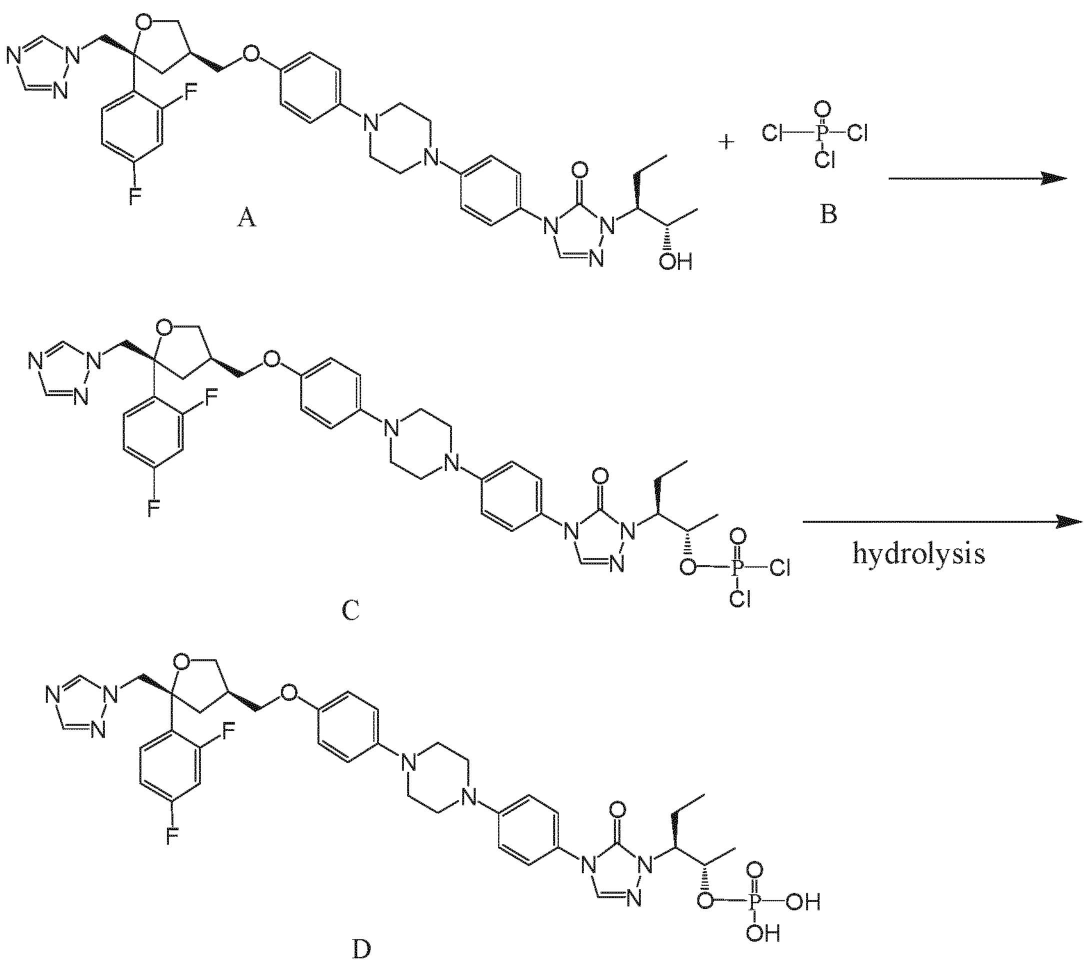


A B C D

40 ml of phosphorus oxychloride was added into a 250 ml dried three-necked flask. The flask was cooled to minus 5 to 5°C under nitrogen protection, and posaconazole (10 g, 14.28 mmol) was slowly added. After completion of the addition, the temperature was maintained and stirring was performed for 12 hours, and the reaction was ended. The progress of the reaction was judged by in-process HPLCC.

Chromatographic conditions are as follows:

Mobile phase: 6.8 g/L monopotassium phosphate whose pH value is adjusted with phosphoric acid to 3.0; ratio of monopotassium phosphate to acetonitrile is 60:40.

Detection wavelength: 220 nm; flow velocity: 1.0 ml/min; column temperature: 25°C Sample concentration: 1 mg/ml, dilution medium: 50% of acetonitrile

The resulting reaction solution was dripped into 800 ml of an aqueous solution of sodium hydroxide at 0°C, the hydrolysis temperature was controlled at 0~5°C. After the hydrolysis was completed, the pH value was adjusted to 3~4 with 10% of hydrochloric acid. Filteration was performed, and the filter cake was washed with acetone and the dried through forced air at 25°C ~ 40°C, and 8.3g of white solid was resulted. Related substances: individual impurity: 0.42%, total impurities: 1.02%.

Step 2:

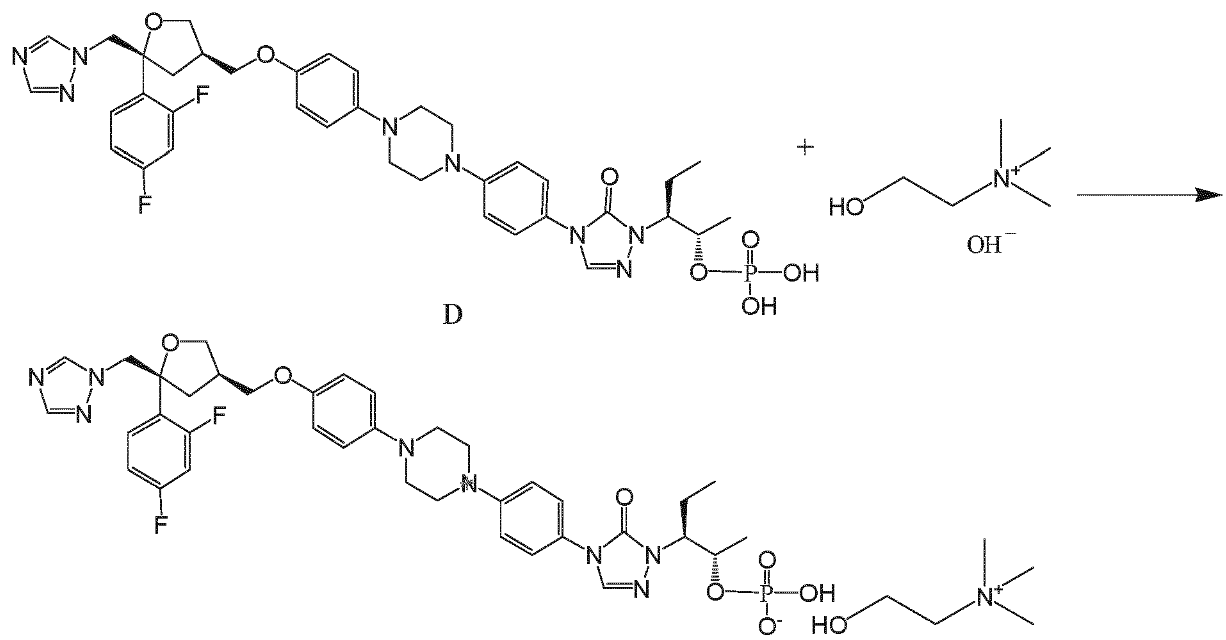

D

Choline hydroxide (0.31 g, 1.28 mmol, 50%) was weighed and added into a beaker filled with 3 ml of purified water, the resulting mixture was stirred uniformly. Compound (formula D) (1 g, 1.28 mmol) was poured into the beaker and stirring was performed at 35°C for 1 hour. After the solid was dissolved, filtration was performed, and the filtrate was poured into a beaker filled with 50 ml of ethanol. The resulting solution was stirrered at 5°C for 12 hours. Suction filtration was performed to yield a white solid which was rinsed with 5 ml of ethanol. The filter cake was dried under vacuum to give 0.76g of white solid (related substances: 0.13%; moisture: 0.3%; content: 99.87%).

**Example 3:** Preparation of posaconazole phosphate monocholinate pentahydrate

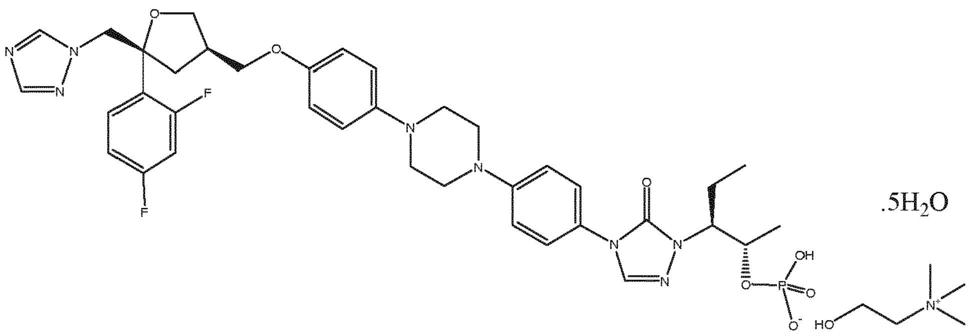

$.5H_2O$

Posaconazole of the same batch as Example 1 was selected to repeat the steps 1 and 2 in Example 2 in order to carry out the experiment, except that a drying manner in the step 2 was changed into forced air drying at 35±5°C. A white solid of the title compound was obtained (related substances: 0.13%; moisture: 9.06%; content: 99.87%).

**Example 4:** Preparation of posaconazole phosphate monocholinate

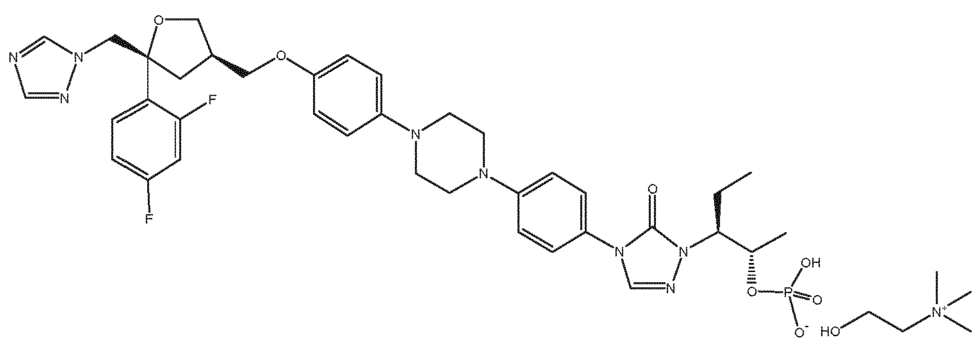

Step 1:

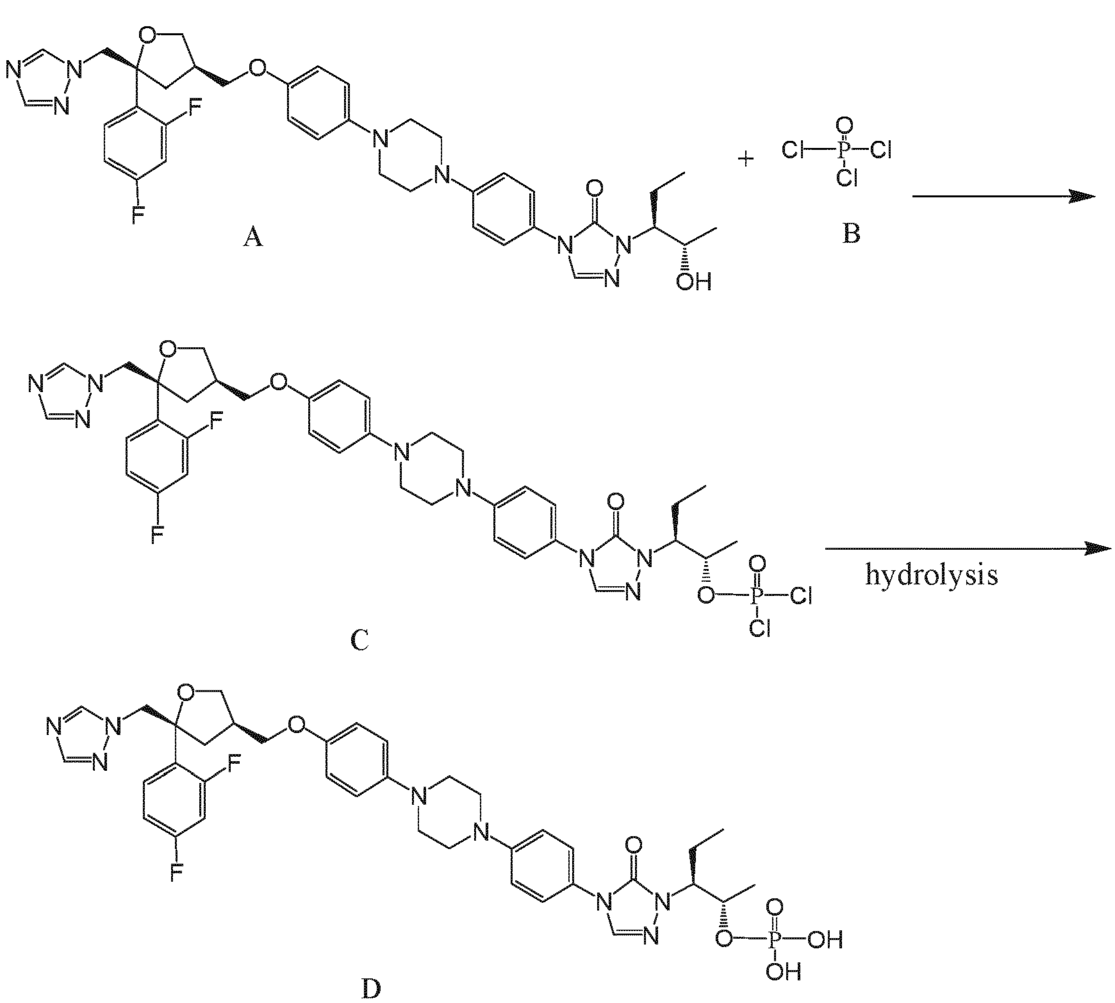


A

B

C

D

Posaconazole (10g, 14.28 mmol) was weighed and added into a 250 ml dried three-necked flask, tetrahydrofuran (20 ml) was added under nitrogen protection, and the resluting solution was stirred to dissolve. Triethylamine (5 ml) was added at 50°C and was stirred, and reaction was carried out at 50°C for 30 minutes. Phosphorus oxychloride (1.3 ml, 14.28 mmol) was slowly added within about 1 minute. The reaction was carried out for 6 hours and the reaction was monitored by HPLC, the result showed that the reaction was about 20% completed. The reaction was continued for 24 hours, and about 81% of the reaction was completed. And reaction was lasted for 36 hours until the reaction was substantially completed. The HPLC chromatographic conditions are the same as those in Example 1.

A reaction solution was dripped into 150 ml of purified water at 10°C, and the hydrolysis temperature was controlled at 10°C. The resulting mixture was stirred for 16 hours. An organic phase and an aqueous phase were

transferred into a separation funnel for extraction. The solid phase was dissolved with MeOH (150 ml) and combined with the extracted organic phase. About 25 g of 200~300 mesh silica gel was poured into the combined phases. The solvent was evaporated to dryness by using a rotary evaporator. The dried silica gel was poured into a column (having a diameter of 4.5 cm) filled with 25cm thick silica gel pad. When the target product was eluted from the column, rotary evaporation was performed to remove the collected solvent so as to yield a yellow solid to which 150 ml of water and 50 ml of dichloromethane were added. Extraction was conducted, and the organic phase was dried over anhydrous $Na_2SO_4$. The dried organic phase was filtered and filtrate was evaporated to dryness by using a rotary evaporator to yield 5.2g of light yellow solid. Related substances: individual impurity: 2.5%, and 4.90% of total impurities: 4.90%.

Step 2:

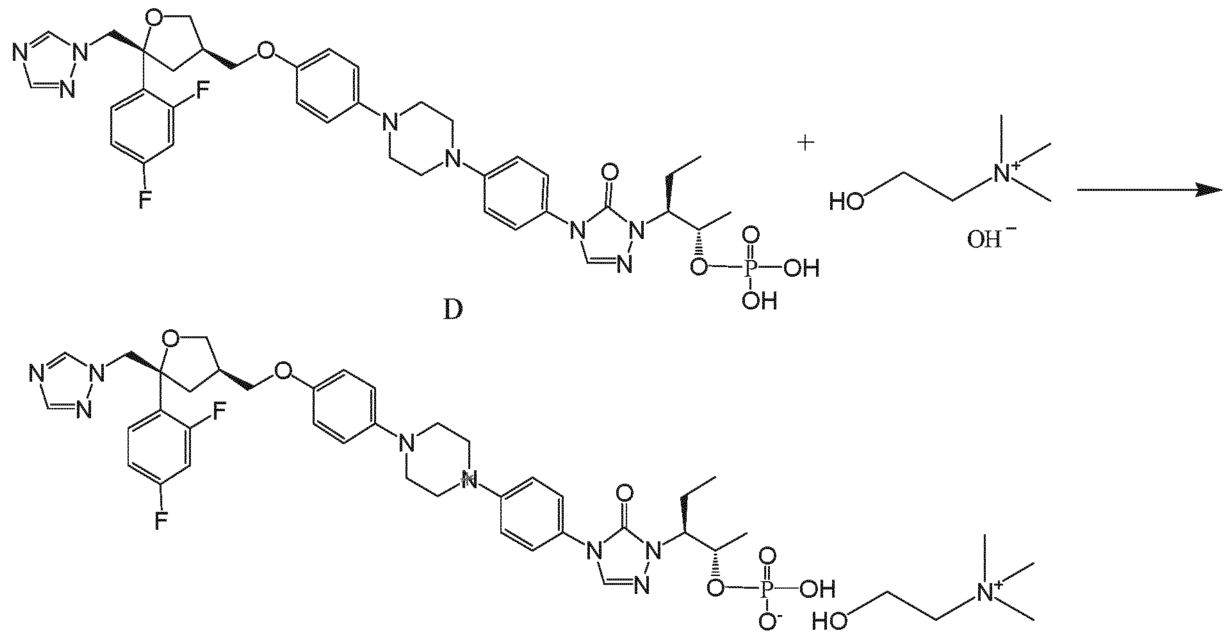

D

Choline hydroxide (0.31 g, 1.28 mmol, 50%) was weighed and added into a beaker filled with 5 ml of purified water, the resulting mixture was stirred uniformly. Compound (formula D) (1 g, 1.28 mmol) was poured into the beaker and stirring was performed at 60°C for 1 hour. After the solid was dissolved, filtration was performed, and the filtrate was poured into a beaker filled with 50 ml of acetone. The resulting solution was stirrered at room temperature for 12 hours. Suction filtration was performed to yield a white solid which was rinsed with 5 ml of acetone. The filter cake was dried under vacuum to give 0.45g of white solid. Related substances: individual impurity: 1.23%, total impurities: 2.54%.

__Example 5:__ Preparation of posaconazole phosphate monocholinate

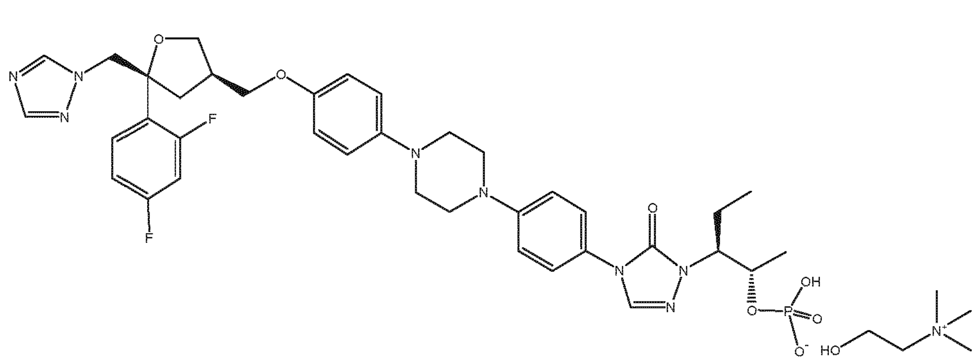

Step 1:

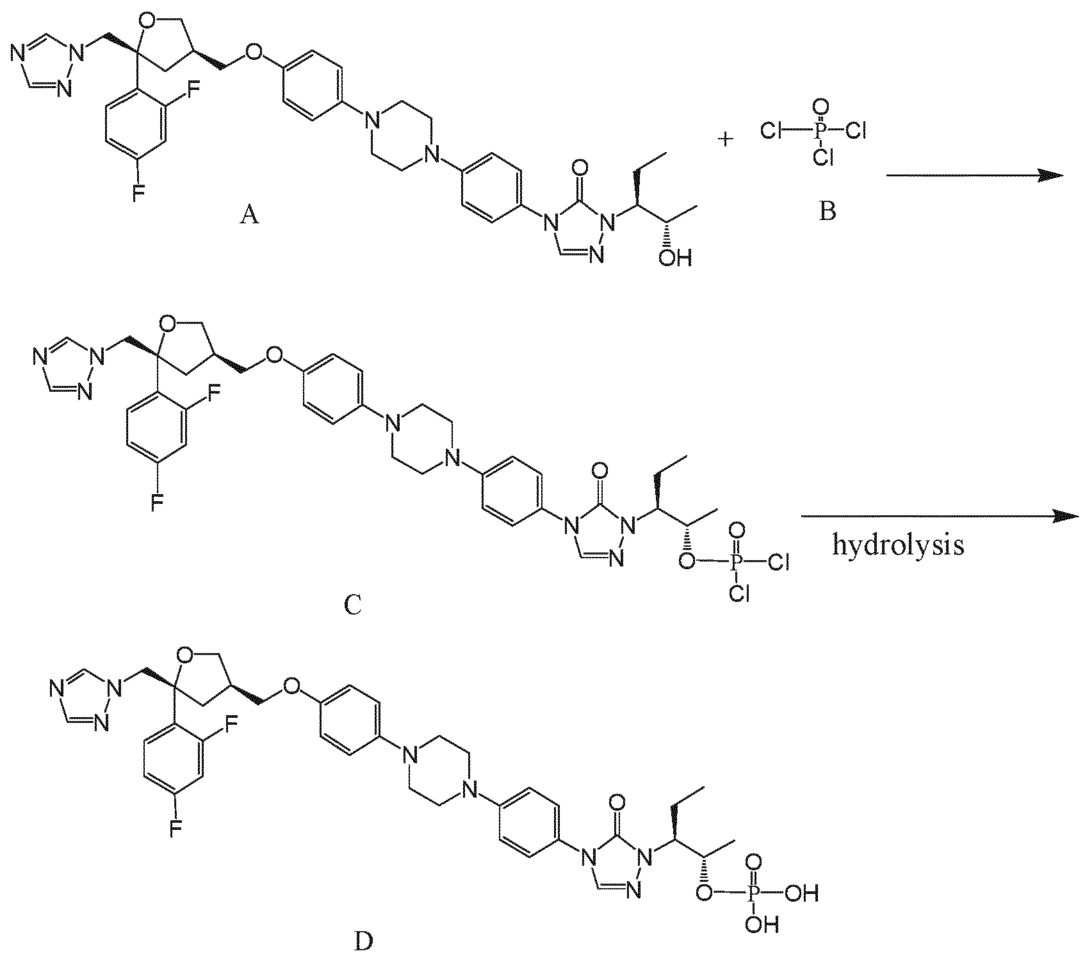


A B C D

Posaconazole (10g, 14.28 mmol) was weighed and added into a dried 250 ml three-necked flask, and acetonitrile (20 ml) was added under nitrogen protection, and stirring was followed to dissolve the solid. Triethylamine (5 ml) was added at 30°C, stirring was performed and the reaction was carried out at 30°C for 30 minutes. Then, phosphorus oxychloride (13 ml, 142.8mmol) was added slowly and the addition was completed within about 1 minute. The resulting mixture was allowed to react for 6 hours, and the reaction was monitored by HPLC, indicating that the reaction was substantially completed. The chromatographic conditions for HPLC were the same as those in Example 1.

A reaction solution was dripped into 150 ml of purified water at 0°C, and the hydrolysis temperature was controlled at minus 5 to 0°C. The resulting mixture was stirred for 16 hours. The organic phase and the aqueous phase were transferred into a separation funnel for extraction. The solid phase was dissolved with MeOH (150 ml) and combined with the extracted organic phase. About 25 g of 200~300-mesh silica gel was poured into the combined phase. The solvent was evaporated to dryness by using a rotary evaporator. The dried silica gel was poured into a column (having a diameter of 4.5 cm) filled with 25cm thick silica gel pad. When the target product was eluted from the column, rotary evaporation was performed to remove the collected solvent to yield a yellow solid to which 150 ml of water and 50 ml of dichloromethane were added. Extraction was performed and the organic phase was dried over anhydrous $Na_2SO_4$ and was evaporated to dryness by using a rotary evaporator to yield 7.3g of light yellow solid. Related substances: individual impurity: 0.26%, and total impurities: 0.68%.

Step 2:

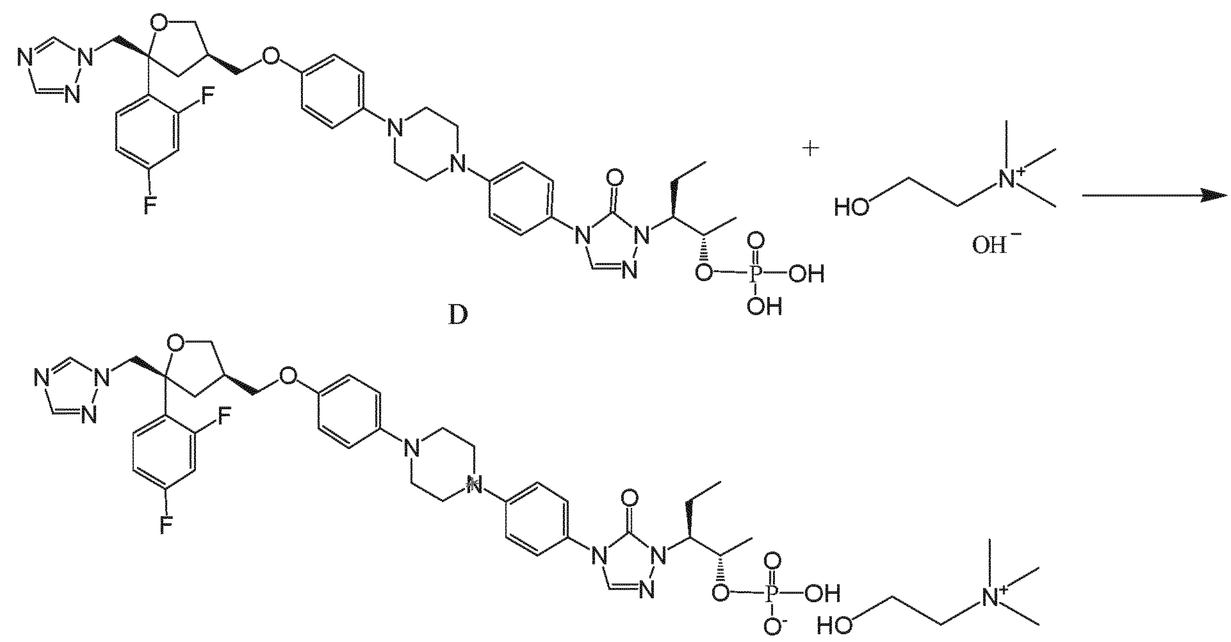

D

Choline hydroxide (0.31 g, 1.28 mmol, 50%) was weighed and added into a beaker filled with 5 ml of purified water, the resulting mixture was stirred uniformly. Compound (formula D) (1 g, 1.28 mmol) was poured into the beaker and stirring was performed at 35°C for 1 hour. After the solid was dissolved, filtration was performed, and the filtrate was poured into a beaker filled with 50 ml of methanol. The resulting solution was stirrered at room temperature for 12 hours. Suction filtration was performed to yield a white solid which was rinsed with 5 ml of methanol. The filter cake was dried under vacuum to give 0.25 g of white solid. Related substances: individual impurity: 0.02%, total impurities: 0.10%.

__**Example 6:**__ Preparation of posaconazole phosphate monocholinate

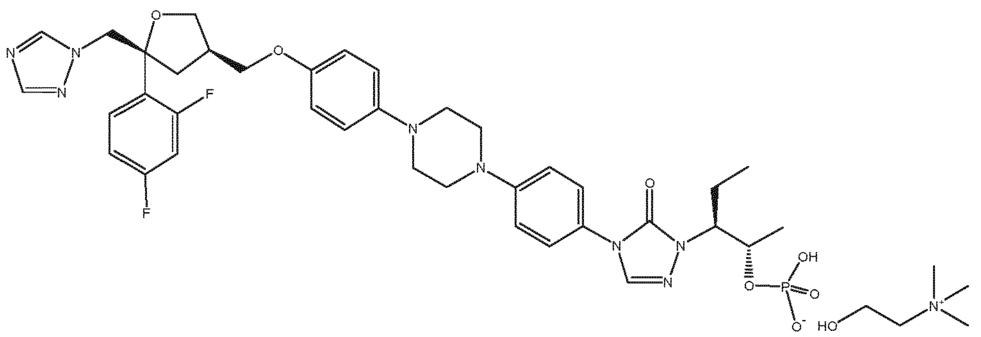

Step 1:

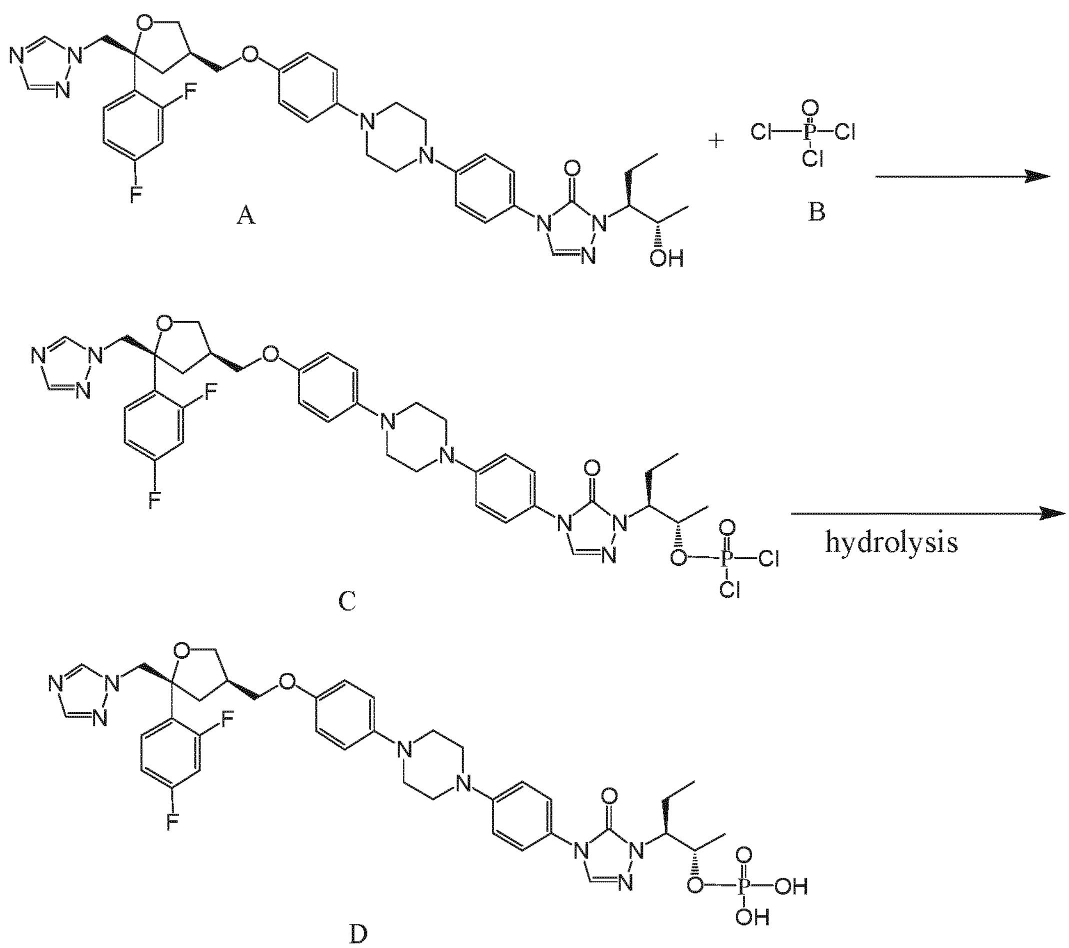


A

B

C

D

Posaconazole (10g, 14.28 mmol) was weighed and added into a dried 250 ml three-necked flask, and chloroform (70 ml) was added under nitrogen protection, and stirring was followed to dissolve. Triethylamine (5 ml) was added at 30°C, stirring was performed and a reaction was carried out at 30°C for 30 minutes. Then, phosphorus oxychloride (6 ml, 64.36mmol) was added slowly and the addition was completed within about 1 minute. The resulting mixture was allowed to react for 6 hours, and the reaction was monitored by HPLC, indicating that the reaction was substantially completed. The chromatographic conditions for HPLC were the same as those in Example 1.

A reaction solution was dripped into 150 ml of purified water at 0°C, and the hydrolysis temperature was controlled at minus 5 to 0°C. The resulting mixture was stirred for 16 hours. An organic phase and an aqueous phase were transferred into a separation funnel for extraction. The solid phase was dissolved with MeOH (150 ml) and combined with the extracted organic phase; about 25 g of 200~300-mesh silica gel was poured into the combined phase. The solvent was evaporated to dryness by using a rotary evaporator. The dried silica gel was poured into a column (having a diameter of 4.5 cm) filled with 25cm thick silica gel pad. When the target product was eluted from the column, rotary evaporation was performed to remove the collected solvent to yield a yellow solid to which 150 ml of water and 50 ml of dichloromethane were added. Extraction was performed and the organic phase was dried over anhydrous $Na_2SO_4$

and was evaporated to dryness by using a rotary evaporator to yield 7.0g of light yellow solid. Related substances: individual impurity: 0.33%, total impurities: 0.61%.

Step 2:

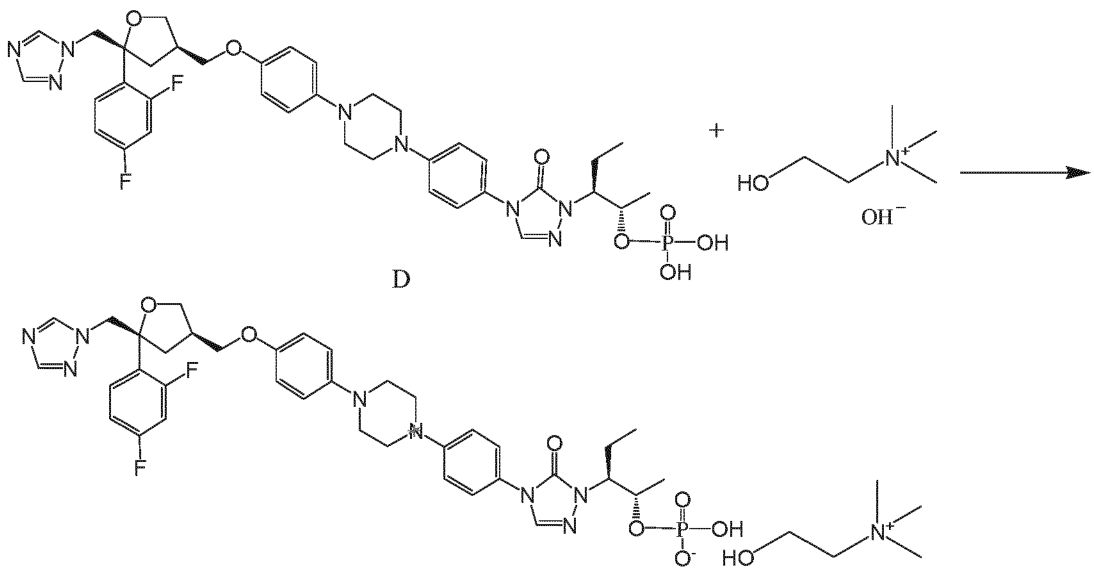

D

Choline hydroxide (0.31 g, 1.28 mmol, 50%) was weighed and added into a beaker filled with 5 ml of purified water, the resulting mixture was stirred uniformly. Compound (formula D) (1 g, 1.28 mmol) was poured into the beaker and stirring was performed at 35°C for 1 hour. After the solid was dissolved, filtration was performed, and the filtrate was poured into a beaker filled with 50 ml of isopropanol. The resulting solution was stirrered at room temperature for 12 hours. Suction filtration was performed to yield a white solid which was rinsed with 5 ml of isopropanol. The filter cake was dried under vacuum to give 0.21 g of white solid. Related substances: individual impurity: 0.22%, total impurities: 0.53%.

<u>**Example 7:**</u> Manufacturing of tablets

Formulation:

| | |
|---|---|
| Posaconazole phosphate monocholinate (prepared in Example 1, calculated as posaconazole) | 50g |
| Starch | 150g |
| Microcrystalline cellulose | 50g |
| 4% povidone K30 | appropriate amount |
| Magnesium stearate | 2.0g |
| 1000 tablets manufactured | |

Maufacturing method: posaconazole phosphate monocholinate was ground and passed through an 80-mesh sieve. Starch in a formulation amount, posaconazole phosphate monocholinate in a formulation amount and microcrystalline cellulose were weighed, and uniformly mixed. The materials were prepared into a soft material with a solution of 4% povidone K30, the soft material was grannulated by using a 20-mesh sieve, the granules were dried at 40~60°C until the moisture in the granules was about 5%. Resifting was performed through a 20-mesh sieve, magnesium stearate in a formulation amount was added, and final mixing was performed. Content of the intermediate was determined. After weight of the tablet was finalized, tablet compression was performed.

**Example 8:** Manufacturing of tablets

Formulation:

| | |
|---|---|
| Posaconazole phosphate monocholinate pentahydrate (prepared in Example 3, calculated as posaconazole) | 50g |
| Starch | 300g |
| Microcrystalline cellulose | 100g |
| 4% povidone K30 | appropriate amount |
| Magnesium stearate | 2.0g |
| 1000 tablets manufactured | |

Maufacturing method: posaconazole phosphate monocholinate pentahydrate was ground and passed through an 80-mesh sieve. Starch in a formulation amount, posaconazole phosphate monocholinate pentahydrate in a formulation amount and microcrystalline cellulose were weighed, and uniformly mixed. The materials were prepared into a soft material with a solution of 4% povidone K30, the soft material was grannulated by using a 20-mesh sieve, the granules were dried at 40~60°C until the moisture in the granules was about 5%. Resifting was performed through a 20-mesh sieve; magnesium stearate in a formulation amount was added, and final mixing was performed. Content of the intermediate was determined. After weight of the tablet was finalized, tablet compression was performed..

**Example 9:** Manufacturing of posaconazole phosphate monocholinate for injection Formulation:

| | |
|---|---|
| Posaconazole phosphate monocholinate pentahydrate (prepared in Example 3, calculated as posaconazole) | 50g |
| Mannitol | 80g |
| Hydrochloric acid | appropriate amount |
| Water for injection | 5L |
| 1000 ampoules manufactured | |

Maufacturing method: water for injection in batch volume was added, and a posaconazole phosphate monocholinate pentahydrate and mannitol were weighed. The materials were fully dissolved by stirring and pH value was adjusted to 5~10 with hydrochloric acid. The resulting solution was filtered through a 0.22μm microfiltration membrane, and filling was performed, and freeze drying, capping and packing were performed.

**Example 10:** Maufacturing of posaconazole phosphate monocholinate for injection Formulation:

| | |
|---|---|
| Posaconazole phosphate monocholinate (prepared in Example 1, calculated as posaconazole) | 50g |
| Glucose | 50g |
| Hydrochloric acid | appropriate amount |
| Water for injection | 5L |
| 1000 ampoules manufactured | |

Maufacturing method: water for injection in batch volume was added, and a posaconazole phosphate monocholinate and glucose were weighed. The materials were fully dissolved by stirring and pH value was adjusted to 5~10

with hydrochloric acid. The resulting solution was filtered through a 0.22μm microfiltration membrane, and filling was performed, and freeze drying, capping and packing were performed.

**Example 11:** Preparation of posaconazole phosphate monocholinate for injection Formulation:

| | |
|---|---|
| Posaconazole phosphate monocholinate pentahydrate (prepared in Example 1, calculated as posaconazole) | 50g |
| Water for injection | 5L |
| 1000 bottles manufactured | |

Maufacturing method: water for injection in batch volume was added, and a posaconazole phosphate monocholinate were weighed. The materials were fully dissolved by stirring. The resulting solution was filtered through a 0.22μm microfiltration membrane, and filling was performed, and freeze drying, capping and packing were performed.

**Comparative example 1:** Preparation of posaconazole phosphate bicholinate

Posaconazole of the same batch as that in Example 1 was selected to conduct the experiment by repeating the steps 1 and 2 in Example 1, except that the molar ratio of a feeding amount of choline hydroxide to a feeding amount of the compound (formula D) in the step 2 was substituted for 2:1, and a title compound as a white solid was obtained.

**Comparative example 2:** Preparation of posaconazole phosphate bicholinate pentahydrate

Posaconazole of the same batch as that in Example 1 was selected to conduct the experiment by repeating the steps 1 and 2 in Example 3, except that the molar ratio of the feeding amount of choline hydroxide to the feeding amount of the compound (formula D) in the step 2 was substituted for 2:1, and a title compound as a white solid was obtained.

**Comparative example 3:** Preparation of posaconazole phosphate monopotassium salt

Posaconazole of the same batch as that in Example 1 was selected to conduct the experiment by repeating the steps 1 and 2 in Example 1, except that the choline hydroxide in the step 2 was substituted for potassium hydroxide and the molar ratio of potassium hydroxide to the compound (formula D) was 1:1. A title compound as a white solid was obtained.

**Comparative example 4:** Preparation of posaconazole phosphate monomeglumine salt

Posaconazole of the same batch as that in Example 1 was selected to conduct the experiment by repeating the steps 1 and 2 in Example 1, except that the choline hydroxide in the step 2 was substituted for meglumine and the molar ratio of meglumine to the compound (formula D) was 1:1. A title compound as a white solid was obtained.

**Comparative example 5:** Preparation of posaconazole phosphate monoarginine salt

Posaconazole of the same batch as that in Example 1 was selected to conduct the experiment by repeating the steps 1 and 2 in Example 1, except that the choline hydroxide in the step 2 was substituted for arginine and the molar ratio of arginine to the compound (formula D) was 1:1. A title compound as a white solid was obtained.

**Comparative example 6:** Preparation of posaconazole phosphate monopotassium salt trihydrate

Posaconazole of the same batch as that in Example 1 was selected to conduct the experiment by repeating the steps 1 and 2 in Example 3, except that the choline hydroxide in the step 2 was substituted for potassium hydroxide and the molar ratio of potassium hydroxide to the compound (formula D) was 1:1. A title compound as a white solid was obtained.

**Comparative example 7:** Preparation of posaconazole phosphate monosodium salt trihydrate Posaconazole of the same batch as that in Example 1 was selected to conduct the experiment by repeating the steps 1 and 2 in Example

3, except that the choline hydroxide in the step 2 was substituted for sodium hydroxide and the molar ratio of sodium hydroxide to the compound (formula D) was 1:1. A title compound as a white solid was obtained.

**Comparative example 8:** Preparation of posaconazole phosphate monosodium salt Posaconazole of the same batch as that in Example 1 was selected to conduct the experiment by repeating the steps 1 and 2 in Example 1, except that the choline hydroxide in the step 2 was substituted for sodium hydroxide and the molar ratio of sodium hydroxide to the compound (formula D) was 1:1. A title compound as a white solid was obtained.

**Comparative example 9:** Preparation of posaconazole phosphate disodium salt pentahydrate Posaconazole of the same batch as that in Example 1 was selected to conduct the experiment by repeating the steps 1 and 2 in Example 3, except that the choline hydroxide in the step 2 was substituted for sodium hydroxide and the molar ratio of sodium hydroxide to the compound (formula D) was 2:1. A title compound as a white solid was obtained.

**Comparative example 10:** Preparation of posaconazole phosphate disodium salt

Posaconazole of the same batch as that in Example 1 was selected to conduct the experiment by repeating the steps 1 and 2 in Example 1, except that the choline hydroxide in the step 2 was substituted for sodium hydroxide and the molar ratio of sodium hydroxide to the compound (formula D) was 2:1. A title compound as a white solid was obtained.

**Comparative example 11:** Preparation of compound of formula E

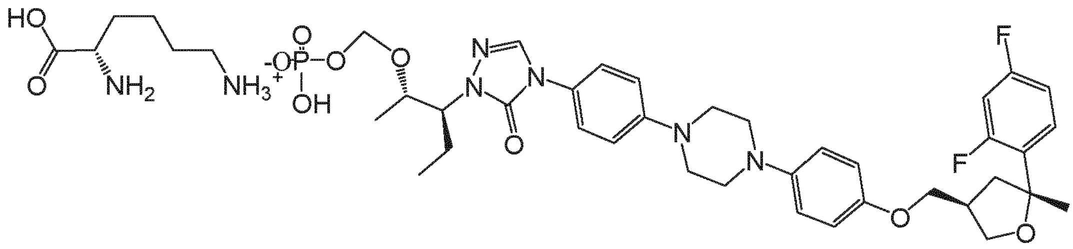

E

Step A: To a 1L round-bottom flask which was dried in a drying oven was equipped with a mechanical stirrer, a nitrogen inlet adapter, a pressure balance addition funnel equipped with a rubber septum, and a temperature probe. Sodium hydride (2.89 g, 0.069 mol, 60%) and THF (50 ml) were added into the round-bottom flask, posaconazole (the compound of formula A) (selected from the same batch of posaconazole as that in Example 1) (17.94 g, 0.023 mol) dissolved in 30 ml of THF was dripped into the stirred suspension within 20 minutes at room temperature. After the solution was stirred for 45 minutes, a solution of iodine (2.99 g, 0.0115 mol) dissolved in 30 ml of THF was dripped within 10 minutes, then di-tert-butyl chloromethyl phosphate (formula III) (13.29 g, 0.035 mol, purity of about 68%) was dripped within 15 minutes. The reaction mixture was stirred at about 41°C for 4 hours to allow the reaction to complete. The progress of the reaction was judged by in-process HPLC.

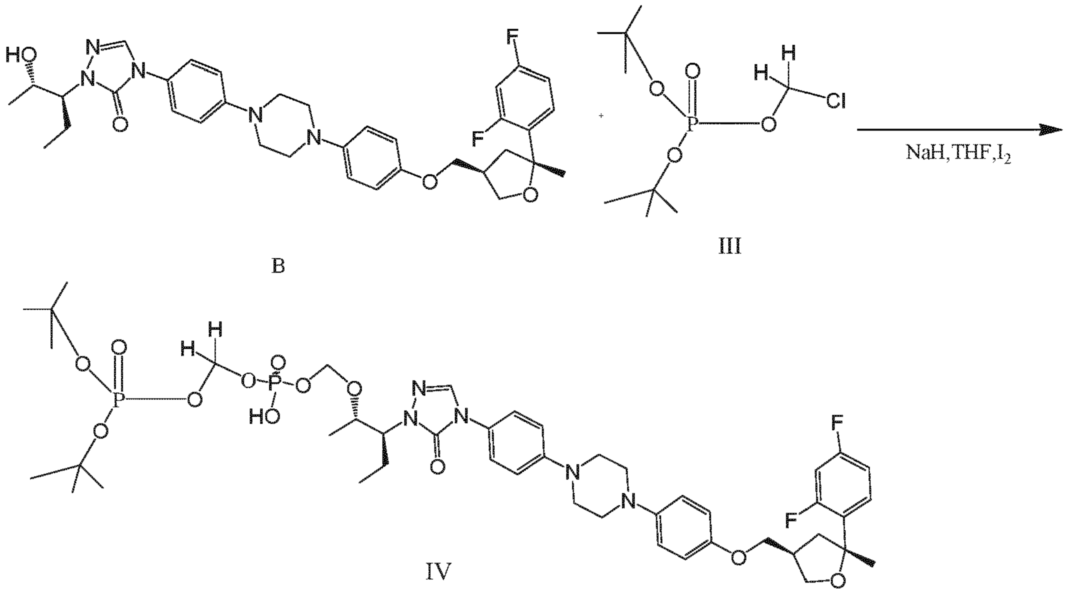


B

III

IV

The reaction mixture was poured into ice water (100 ml). The aqueous phase was separated and extracted with ethyl acetate (3×50ml), the mixed organic extracts was washed with 10% of sodium thiosulfite (50 ml), water (50 ml) and brine (50 ml); the organic phase was dried over magnesium sulfate concentrated under vacuum, giving a light yellow oily matter (22.8 g, in the process, a reaction endpoint was established when the tracked product reaction degree was up to 97% or higher by HPLC). The crude product (formula IV) ws used in the step B as an initial state.

Step B: The round-bottom flask was equipped with a magnetic stirrer, a cooling bath, a pH probe and a nitrogen inlet/outlet. The product (formula IV) (7.5 g) in the step A, dissolved in $CH_2Cl_2$ (23ml), was added into the round-bottom flask. The resulting solution was cooled to 0°C, and trifluoroacetic acid (8.8 ml) was slowly added into the stirred solution. Then the solution was stirred for 3 hours until the reaction was completed. The progress of the reaction was judged by in-process HPLC. The reaction mixture was poured into a cooled solution of 2N NaOH (64 ml). The reaction mixture was extracted with tert-butyl acetate (2×65ml) to remove all organic impurities; the water layer containing a disodium salt product was treated with activated carbon (10 g) and filtered through a diatomite layer. The clear filtrate was acidized with IN HCl to reach a pH value of 2.5. A free acid product was extracted in ethyl acetate (2×50ml), the combined organic layer was washed with water, dried over $MgSO_4$, and filtered. The filtrate was concentrated under reduced pressure to give 3.39g of crude product of Formula V.

Optionally, in a preferred aspect of the present invention, the step B may be continuously performed. Details of the step B may be determined by those ordinary skilled in the art.

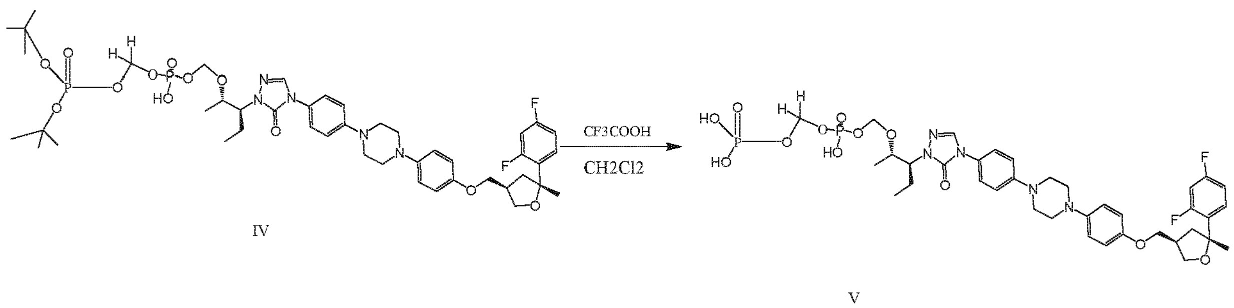


IV

V

Step C: the above product of Formula V was dissolved in methanol (75 ml). L-lysine (1.8 g) was added into the free acid solution, and the pH value was maintained at 4.2~5.5. The resulting mixture was heated at 60°C for 4.5 hours. The hot reactants were filtered through a diatomite layer. The filtrate was concentrated to about 5 ml, combined with ethanol (100 ml) and heated to 65°C so as to crystallize out a solvate of monolysine salt. The solvate was collected onto a Buchner funnel, and dried under vacuum, giving 3.0g of the crystalline solid of title solvate compound.

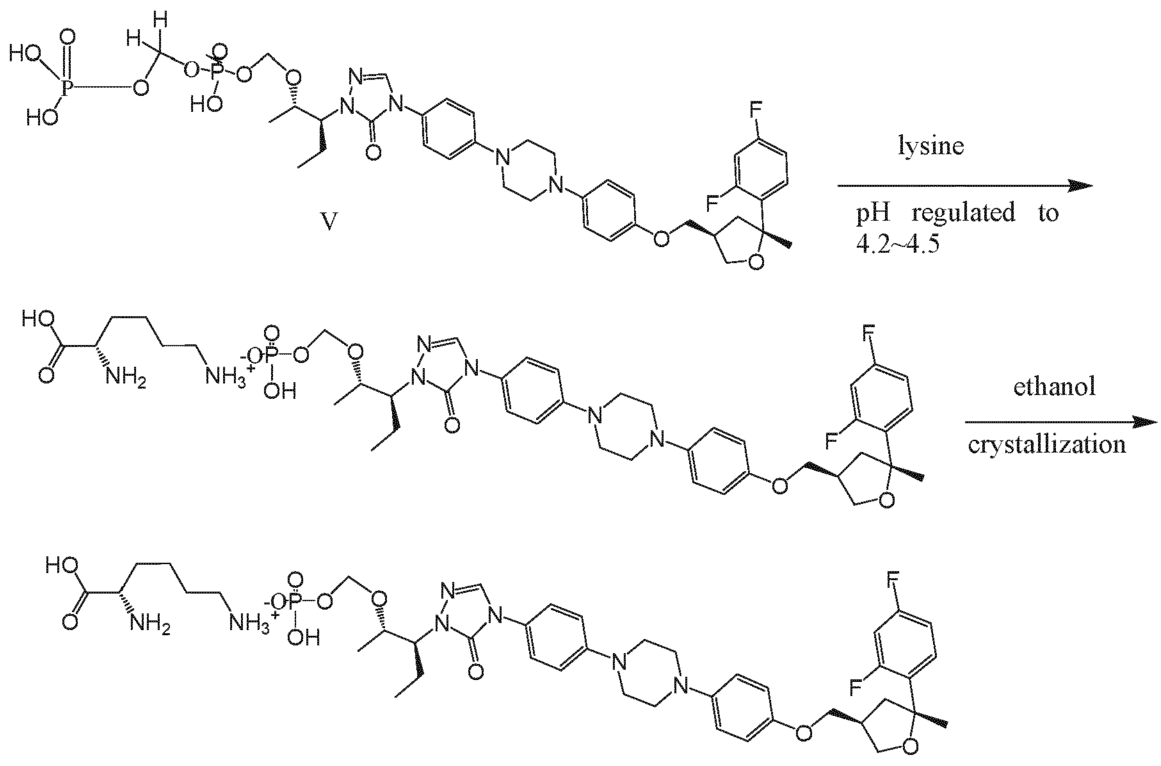


V

**Experiment 1:** Solubility study

The compound obtained in the present invention, the compound obtained in Comparative examples 1~11 and posaconazole were tested for solubility in water, isopropanol and methanol, respectively. Test results are shown in the following table.

Solubility data of tested compounds

| Compound | Water | Methanol | Isopropanol |
|---|---|---|---|
| Example 1 | 82mg/ml | 35mg/ml | Img/ml |
| Example 3 | 84mg/ml | 36mg/ml | 1mg/ml |
| Comparative Example 1 | 76mg/ml | 15mg/ml | 0.7mg/ml |
| Comparative Example 2 | 78mg/ml | 19mg/ml | 0.9mg/ml |
| Comparative Example 3 | 34mg/ml | 14mg/ml | 0.6mg/ml |
| ComparativeExample 4 | 63mg/ml | 10mg/ml | 0.4mg/ml |
| Comparative Example 5 | 71mg/ml | 11mg/ml | 0.7mg/ml |
| Comparative Example 6 | 35mg/ml | 15mg/ml | Img/ml |
| Comparative Example 7 | 67mg/ml | 8mg/ml | 0.3mg/ml |
| Comparative Example 8 | 68mg/ml | 9mg/ml | 0.4mg/ml |
| Comparative Example 9 | 72mg/ml | 10mg/ml | 0.8mg/ml |
| Comparative Example 10 | 72mg/ml | 10mg/ml | 0.8mg/ml |
| Comparative Example 11 | 70mg/ml | 14mg/ml | Img/ml |
| Posaconazole | 0.05mg/ml | 0.06mg/ml | 0.1mg/ml |

It can be seen from experimental results that the compound prepared in the present invention has high solubility

that is higher than that of the compound obtained in Comparative examples 1~11 and than that of the posaconazole in water, methanol and isopropanol. Given the same drug loading capacity, the solubility of the compound of the present invention is higher than the solubility of the compound obtained in the Comparative examples 1~11 and that of the posaconazole. Therefore, the compound of the present invention has fast action and high bioavailability, and the formulation stability is increased. Thus, the compound prepared in the present invention is of great significance to improve its bioavailability and therapeutic effects.

**Experiment 2:** Hygroscopicity study

By referring to *9103 Drug Hygroscopicity Test Guideline Of General Rules Of 2015 Chinese Pharmacopoeia (Part Fourth),* hygroscopicity of the compound in the present application and the compounds in the Comparative examples was investigated.

Test method:

1. A dried glass weighing bottle with stopper (having an outer diameter of 50 mm and a height of 15 mm) was placed in a thermostatic drier (a saturated ammonium chloride solution was placed on the lower part of the thermostatic drier) at an appropriate temperature of 25°C±1°C on the day earlier before the test, and the weight ($m_1$) of weighing bottle was precisely weighed.
2. An appropriate amount of test sample was taken and spread in the weighing bottle, and the thickness of the test sample was controlled to about 1 mm, and the weight ($m_2$) was precisely weighed.
3. The weighing bottle was opened and was placed, togetther with the bottle cap, under the above mentioned constant temperature and humidity condition for 24 hours.
4. The weighing bottle was capped and precisely weighed to obtain the weight ($m_3$).

$$\text{Weight gain percentage} = \frac{m_3 - m_2}{m_2 - m_1} \times 100\%$$

Description of hygroscopicity characterization and delimitation of hygroscopicity weight gain:

Deliquescence: enough moisture is absorbed to form a liquid.
Very hygroscopic: hygroscopic weight gain is not less than 15%.
Moderately Hygroscopic: hygroscopic weight gain is less than 15% but not less than 2%.
Slightly hygroscopic: hygroscopic weight gain is less than 2% but not less than 0.2%.
Not or hardly hygroscopic: hygroscopic weight gain is less than 0.2%.

Hygroscopicity test results of the product after 24 hours are shown in the following table.

Hygroscopicity test results of the tested compounds

| Compound | Hygroscopic weight gain | Conclusion |
|---|---|---|
| Example 1 | 0.12% | No hygroscopicity |
| Example 3 | 0.15% | No hygroscopicity |
| Comparative example 1 | 8.76% | Hygroscopicity |
| Comparative example 2 | 11.81% | Hygroscopicity |
| Comparative example 3 | 1.56% | Slight hygroscopicity |
| Comparative example 4 | 0.88% | Slight hygroscopicity |
| Comparative example 5 | 1.02% | Slight hygroscopicity |
| Comparative example 6 | 1.95% | Slight hygroscopicity |
| Comparative example 7 | 1.86% | Slight hygroscopicity |
| Comparative example 8 | 1.92% | Slight hygroscopicity |
| Comparative example 9 | 12.01% | Hygroscopicity |

(continued)

| Compound | Hygroscopic weight gain | Conclusion |
|---|---|---|
| Comparative example 10 | 13.91% | Hy groscopicity |
| Comparative example 11 | 0.76% | Slight hygroscopicity |

**Experiment 3:** Stability study

1. Stability experiments conducted under conditions of 25°C±2°C and 65% R.H±5% R.H In the present experiment, the posaconazole phosphate monocholinate of the present invention and the compounds in the Comparative examples are subjected to stability experiments under the conditions of 25°C±2°C and 65% R.H±5% R.H. Sampling was performed to determine descriptions, related substances and content at month 0, month 3, month 6, month 9, month 12 and month 24. The results are shown in detail in the following table.

| Tim e | Item | Example 1 | Example 3 | Comparative Exam-ple 1 | Comparative Exam-ple 2 | Comparative Exam-ple 3 | Comparative Exam-ple 4 | Comparative Exam-ple 5 | Comparative Exam-ple 6 | Comparative Exam-ple 7 | Comparative Exam-ple 8 | Comparative Exam-ple 9 | Comparative Exam-ple 10 | Comparative Exam-ple 11 |
|---|---|---|---|---|---|---|---|---|---|---|---|---|---|---|
| month 0 | Description | White powder | White powder | White powder | White powder | White powder | White powder | White powder | White powder | White powder | White powder | White powder | White powder | White powder |
| | Related substance | 0.13% | 0.13% | 0.13% | 0.13% | 0.13% | 0.13% | 0.13% | 0.13% | 0.13% | 0.13% | 0.13% | 0.13% | 0.13% |
| | Content | 99.87% | 99.87% | 99.87% | 99.87% | 99.87% | 99.87% | 99.87% | 99.87% | 99.87% | 99.87% | 99.87% | 99.87% | 99.87% |
| month 3 | Description | White powder | White powder | White powder | White powder | White powder | White powder | White powder | White powder | White powder | White powder | White powder | White powder | White powder |
| | Related substance | 0.13% | 0.13% | 0.14% | 0.14% | 0.20% | 0.19% | 0.21% | 0.22% | 0.22% | 0.27% | 0.27% | 0.28% | 0.28% |
| | Content | 99.87% | 99.87% | 99.86% | 99.86% | 99.80% | 99.81% | 99.79% | 99.78% | 99.78% | 99.73% | 99.73% | 99.72% | 99.72% |
| month6 | Description | White powder | White powder | White powder | White powder | White powder | White powder | White powder | White powder | White powder | White powder | White powder | White powder | White powder |
| | Related substance | 0.13% | 0.13% | 0.19% | 0.19% | 0.32% | 0.31% | 0.29% | 0.30% | 0.29% | 0.37% | 0.32% | 0.38% | 0.39% |
| | Content | 99.87% | 99.87% | 99.81% | 99.81% | 99.68% | 99.69% | 99.71% | 99.70% | 99.71% | 99.67% | 99.68% | 99.62% | 99.61% |
| month 9 | Description | White powder | White powder | White powder | White powder | White powder | White powder | White powder | White powder | White powder | White powder | White powder | White powder | White powder |
| | Related substance | 0.15% | 0.16% | 0.23% | 0.25% | 0.41% | 0.43% | 0.42% | 0.41% | 0.39% | 0.45% | 0.49% | 0.43% | 0.41% |
| | Content | 99.85% | 99.84% | 99.77% | 99.75% | 99.59% | 99.57% | 99.58% | 99.59% | 99.61% | 99.55% | 99.51% | 99.57% | 99.59% |
| month 12 | Description | White powder | White powder | White powder | White powder | White powder | White powder | White powder | White powder | White powder | White powder | White powder | White powder | White powder |
| | Related substance | 0.16% | 0.16% | 0.31% | 0.29% | 0.52% | 0.50% | 0.51% | 0.52% | 0.49% | 0.59% | 0.59% | 0.60% | 0.60% |

(continued)

| Tim e | Item | Example 1 | Example 3 | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 |
|---|---|---|---|---|---|---|---|---|---|---|---|---|---|---|
| | Content | 99.84% | 99.84% | 99.61% | 99.71% | 99.48% | 99.50% | 99.49% | 99.48% | 99.51% | 99.41% | 99.41% | 99.40% | 99.40% |
| month 24 | Description | White powder | White powder | White powder | White powder | White powder | White powder | White powder | White powder | White powder | White powder | White powder | White powder | White powder |
| | Related substance | 0.20% | 0.21% | 0.78% | 0.82% | 0.97% | 1.02% | 0.97% | 1.00% | 1.02% | 1.12% | 1.13% | 1.16% | 1.14% |
| | Content | 99.80% | 99.79% | 99.22% | 99.18% | 99.03% | 98.98% | 99.03% | 99.00% | 98.98% | 98.88% | 98.87% | 98.84% | 98.86% |

It can be seen from experimental results that stability of the posaconazole phosphate monocholinate is higher than that of the compounds in Comparative examples 1~11, which facilitates storage in industrial production.

2. Stability experiments conducted under conditions of 40°C±2°C and 75% R.H±5% R.H The posaconazole phosphate monocholinate and the compounds in the Comparative examples are subjected to stability experiments under the conditions of 40°C±2°C and 75% R.H±5% R.H. Sampling was performed to determine descriptions, related substances and content at month 0, month 1, month 2, month 3, and month 6. The results are shown in detail in the following table.

| Time | Item | Example 1 | Example 3 | Comparative example 1 | Comparative example 2 | Comparative example 3 | Comparative example 4 | Comparative example 5 | Comparative example 6 | Comparative example 7 | Comparative example 8 | Comparative example 9 | Comparative example 10 | Comparative example 11 |
|---|---|---|---|---|---|---|---|---|---|---|---|---|---|---|
| month 0 | Description | White powder | White powder | White powder | White powder | White powder | White powder | White powder | White powder | White powder | White powder | White powder | White powder | White powder |
| | Related substance | 0.13% | 0.13% | 0.13% | 0.13% | 0.13% | 0.13% | 0.13% | 0.13% | 0.13% | 0.13% | 0.13% | 0.13% | 0.13% |
| | Content | 99.87% | 99.87% | 99.87% | 99.87% | 99.87% | 99.87% | 99.87% | 99.87% | 99.87% | 99.87% | 99.87% | 99.87% | 99.87% |
| month 1 | Description | White powder | White powder | White powder | White powder | White powder | White powder | White powder | White powder | White powder | White powder | White powder | White powder | White powder |
| | Related substance | 0.13% | 0.13% | 0.14% | 0.14% | 0.25% | 0.27% | 0.28% | 0.24% | 0.23% | 0.29% | 0.28% | 0.28% | 0.29% |
| | Content | 99.87% | 99.87% | 99.86% | 99.86% | 99.75% | 99.73% | 99.72% | 99.76% | 99.77% | 99.71% | 99.72% | 99.72% | 99.71% |
| month 2 | Description | White powder | White powder | White powder | White powder | White powder | White powder | White powder | White powder | White powder | White powder | White powder | White powder | White powder |
| | Related substance | 0.14% | 0.13% | 0.16% | 0.16% | 0.36% | 0.38% | 0.40% | 0.35% | 0.37% | 0.37% | 0.38% | 0.40% | 0.40% |
| | Content | 99.86% | 99.87% | 99.84% | 99.85% | 99.64% | 99.62% | 99.60% | 99.65% | 99.63% | 99.63% | 99.62% | 99.60% | 99.60% |
| month 3 | Description | White powder | White powder | White powder | White powder | White powder | White powder | White powder | White powder | White powder | White powder | White powder | White powder | White powder |
| | Related substance | 0.15% | 0.14% | 0.20% | 0.21% | 0.49% | 0.50% | 0.50% | 0.51% | 0.50% | 0.53% | 0.55% | 0.55% | 0.55% |
| | Content | 99.85% | 99.86% | 99.80% | 99.79% | 99.51% | 99.50% | 99.50% | 99.49% | 99.50% | 99.47% | 99.45% | 99.45% | 99.45% |

(continued)

| Time | Item | Example 1 | Example 3 | Comparativ e example 1 | Comparativ e example 2 | Comparativ e example 3 | Comparativ e example 4 | Comparativ e example 5 | Comparativ e example 6 | Comparativ e example 7 | Comparativ e example 8 | Comparativ e example 9 | Comparativ e example 10 | Comparative example 11 |
|---|---|---|---|---|---|---|---|---|---|---|---|---|---|---|
| | Description | White powder | White powder | White powder | White powder | White powder | White powder | White powder | White powder | White powder | White powder | White powder | White powder | White powder |
| month 6 | Related substance | 0.16% | 0.15% | 0.35% | 0.36% | 0.62% | 0.64% | 0.63% | 0.62% | 0.63% | 0.68% | 0.68% | 0.66% | 0.66% |
| | Content | 99.84% | 99.85% | 99.65% | 99.64% | 99.38% | 99.36% | 99.37% | 99.38% | 99.37% | 99.32% | 99.32% | 99.34% | 99.34% |

Conclusion: after an accelerated test of 6 months, various inspection indexes of the posaconazole phosphate monocholinate prepared in the present invention showes no significant changes compared with those at month 0. However, after an accelerated test of 6 months, when comparing various inspection indexes of the compounds in the Comparative examples 1~11 with those at month 0, related substances increased significantly, which indicates that the stability of the posaconazole phosphate monocholinate of the present invention is higher than that of the compounds in the Comparative examples 1~11.

**Experiment 4:** Test on affecting factors

The posaconazole phosphate monocholinate invented in the present invention was placed under conditions of high temperature of 40°C, illumination of 4500 LX and high humidity of 92.5% for 10 days, and sampling was performed to detect descriptions, related substances and content on day 0, day 5 and day 10. Results are shown in the following table.

Result of affecting factor test

| | Time | Condition | Description | Related sub stance | Content |
|---|---|---|---|---|---|
| Exampl e 1 | day 0 | - | White powder | 0.13% | 99.87% |
| | day 5 | High temperature of 40°C | White powder | 0.13% | 99.87% |
| | | High humidity of 92.5% | White powder | 0.14% | 99.86% |
| | | Illumination of 4500LX | White powder | 0.13% | 99.87% |
| | day 10 | High temperature of 40°C | White powder | 0.15% | 99.85% |
| | | High humidity of 92.5% | White powder | 0.14% | 99.86% |
| | | Illumination of 4500LX | White powder | 0.14% | 99.86% |
| Exampl e 3 | Time | Condition | Description | Related substance | Content |
| | day 0 | - | White powder | 0.13% | 99.87% |
| | day 5 | High temperature of 40°C | White powder | 0.13% | 99.87% |
| | | High humidity of 92.5% | White powder | 0.14% | 99.86% |
| | | Illumination of 4500LX | White powder | 0.13% | 99.87% |
| | day 10 | High temperature of 40°C | White powder | 0.15% | 99.85% |
| | | High humidity of 92.5% | White powder | 0.14% | 99.86% |
| | | Illumination of 4500LX | White powder | 0.14% | 99.86% |

Conclusion: after being placed under the conditions (the high temperature, high humidity and illumination) for 10 days, various inspection indexes of the compound prepared in the present invention shows no significant changes compared with those at day 0, indicating that the compound of the present invention has stable properties.

**Experiment 5:** Experiment of intravenous administration of the compound of the present invention to resist candida albicans vaginitis

1. Experimental materials

1.1 Experimental apparatuses

Blood cell counting plate, paraffin slicing machine, SPX-250B biochemical incubator, untraclean bench, Finnpipette, pressurized steam sterilizer, optical microscope and electronic analytical balance.

1.2 Experiment reagents

Estradiol benzoate injection, polyethylene glycol and Sabouraud glucose solid agar culture medium

1.3 Experimental animal

KM mice, body weight of 18~22 g, female, provided by Jiangsu Experimental Animal Center 1.4 Experimental strain

Standard strain of candida albicans was purchased from American Type Culture Collection center, the strain number was ATCC10231.

2. Experimental method

The above mice were weighed and randomly assigned to groups: posaconazole group, test compound group and solvent group. Each group included 20 mice. Since the posaconazole is insoluble in the solvent (normal saline), a commercially available posaconazole injection (MSD/Schering-Plough, 3PAR80701, same hereinafter) was used in the the posaconazole group in the experiment. That was, sulfobutyl ether-β-cyclodextrin was used for solubilizing. Before infected by candida albicans, animals in each group were continuously subjected to subcutaneous injection with 0.5 ml of estradiol benzoate (2 mg/ml) for 6 days to allow the animals progress into estrus. Later, the animals were injected once every 2 days until the experiment was ended. Six (6) days later, 20 μl of candida albicans liquid having a concentration of 3.5 × $10^6$CFU/ml was injected into the vagina of each mouse, thereby creating a vaginal infection model. From the first day after infection, corresponding drugs in 20 mg/kg (calculated as posaconazole) were administered to the caudal veins of the animals in each group. The dosing volume was 0.1 mg/kg, once every day, drug administration was performed for 5 consecutive days, and the model group was administered with the solvent of the equal volume (normal saline). On day 3 and day 5 after the mice was infected, the vagina of the mice were rubbed by sterile swabs, the swabs were soaked into 0.9 ml of normal saline, and the bacteria liquid was diluted into series of concentrations by a 10-times increment, and 100 μl of the bacteria liquid of each concentration was respectively taken and inoculated with Sabouraud glucose solid agar culture medium containing 0.5% (W/V) chloramphenicol, and fungal loading capacity of the candida albicans in the vagina was observed.

3. Experimental results

Candida albicans vaginitis (intravenous administration): fungal loading capacity of the vagina of the mice in each group

| Group | Dose (mg/kg) | Time (day) | |
|---|---|---|---|
| | | 3 | 5 |
| Solvent group | - | 4.08±0.36 | 4.48±0.52 |
| Posaconazole | 20 | 4.08±0.25 | 3.90±0.22 |
| Example 7 | 20 | 4.11±0.30 | 3.85±0.28 |
| Example 8 | 20 | 4.08±0.52 | 3.87±0.31 |
| Example 9 | 20 | 4.05±0.25 | 3.86±0.21 |
| Note: the data is represented by the mean of logarithm of CFU value for 20 mice +/- standard deviation. | | | |

It can be seen from the experimental results that 5 days after intravenous administration, the fungal loading capacity in the mice in the group of the compound of the present invention was significantly decreased compared with that in the solvent group, and is consistent with that in the posaconazole group, and obvious therapeutic effects are achieved. Moreover, safety risk brought by use of sulfobutyl ether-β-cyclodextrin for solubilizing is avoided.

**Experiment 6:** Experiment of intragastric administration of the compound of the present invention to resist candida albicans vaginitis

1. Experimental materials

1.1 Experimental apparatuses

Blood cell counting plate, paraffin slicing machine, SPX-250B biochemical incubator, untraclean bench, Finnpipette, pressurized steam sterilizer, optical microscope and electronic analytical balance.

1.2 Experiment reagents

Estradiol benzoate injection, polyethylene glycol and Sabouraud glucose solid agar culture medium

1.3 Experimental animal

KM mice, body weight of 18~22 g, female, provided by Jiangsu Experimental Animal Center 1.4 Experimental strain

Standard strain of candida albicans was purchased from American Type Culture Collection center, strain number ATCC10231.

2. Experimental method

The above mice were weighed and randomly assigned to groups: posaconazole group (CMC-Na), test compound group and solvent group. Each group included 20 mice. Since the posaconazole is insoluble in the solvent (normal saline), a commercially available posaconazole injection (MSD/Schering-Plough, 3PAR80701, same hereinafter) was used in the posaconazole group in the experiment. That was, sulfobutyl ether-β-cyclodextrin was used for solubilizing. Other tested drugs were dissolved with the normal saline and subjected to ultrasonic treatment until it became clear so as to be used for administration. Before infected by candida albicans, animals in each group were continuously subjected to subcutaneous injection with 0.5 ml of estradiol benzoate (2 mg/ml) for 6 days to allow the animals progress into estrus. Later, the animals were injected once every 2 days until the experiment was ended. Six (6) days later, 20 μl of candida albicans liquid having a concentration of $3.5 \times 10^6$CFU/ml was injected into the vagina of each mouse, thereby creating a vaginal infection model. From the first day after infection, corresponding drugs in 20 mg/kg (calculated as posaconazole) were intragastrically administered to the animals in each group. The dosing volume was 0.1 mg/kg, once every day, drug administration was performed for 15 consecutive days, and the model group was administered with the solvent of the equal volume (normal saline). On Day 3, Day 5, Day 7 and 15 after the mice in each group was infected, the vagina of the mice were wiped by sterile swabs, the swabs were soaked into 0.9 ml of normal saline, and the bacteria liquid was diluted into series of concentrations by a 10-times increment; and 100 μl of the bacteria liquid of each concentration was respectively taken and inoculated with Sabouraud glucose solid agar culture medium containing 0.5% (W/V) chloramphenicol, and fungal loading capacity of the candida albicans in the vagina was observed.

3. Experimental results

Candida albicans vaginitis (intragastric administration): fungal loading capacity of the vaginas of the mice in each group

| Group | Dose (mg/kg) | Time (day) | | | | |
|---|---|---|---|---|---|---|
| | | 3 | 5 | 7 | 11 | 15 |
| Solvent group | - | 4.24±0.31 | 4.39±0.48 | 5.23±0.41 | 5.45±0.39 | 5.87±0.65 |
| Posaconazole | 20 | 4.21±0.34 | 4.29±0.58 | 4.15±0.38 | 3.78±0.31 | 2.95±0.22 |
| Example 10 | 20 | 4.18±0.22 | 4.35±0.41 | 3.97±0.47 | 3.05±0.27 | 2.29±0.19 |

It can be seen from the experimental results that 15 days after administration, the fungal loading capacity in the mice in the group of the compound, in which normal saline was used to dissovle the compound of the present invention was significantly decreased compared with that in the solvent group, and is consistent with that in the posaconazole group, and obvious therapeutic effects are achieved.

**Experiment 7:** Experiment of intravenous administration of the compound of the present invention to resist systemic fungal infection in mice

1. Experimental material

1.1 Experimental apparatuses

Multiskan MK3 ELISA detector, waterproof electro-heating standing-temperature cultivator, zo-F160 whole

temperature shaking incubator, MJX intelligent mold incubator, SW-CT-IF ultraclean bench and ultraviolet spectrophotometer

1.2 Experiment reagent

Dimethyl sulfoxide and Sabouraud glucose solid agar culture medium (SDA)

1.3 Experimental animal

ICR mice, body weight of 18~22 g, female, provided by Hubei Experimental Animal Center

1.4 Experimental strain

Standard strain of candida albicans was purchased from American Type Culture Collection center, the strain number was ATCC10231.

2. Experimental method

Before the experiment, a small amount of candida albicans was picked by an inoculation loop from the SDA medium (Sabouraud agar, same hereinafter) which was preserved at 4°C, the candida albicans was inoculated to 1 ml of YPD (Yeast Extract Peptone Dextrose Medium) culture solution. The culture was vibrated at 200 rpm at 30°C. The fungi were activated for 16 hours and allowed to be enter a later phase of an exponential growth phase. The fungi were counted by using a blood cell counting plate, and the concentration of the bacteria liquid was adjusted to $1 \times 10^3$~$5 \times 10^3$CFU/ml with RPMI1640 (RoswellPark Memorial Institute1640, same hereinafter). The candida albicans monoclone on the SDA plate was picked and was cultured at 200 rpm at 35°C to reach the later phase of the exponential growth phase, and inoculated to 1 ml of YPD (Yeast Extract Peptone Dextrose Medium, similarly hereinafter), 1% of the culture was inoculated to a fresh medium and cultured for 6 hours, centrifugation was performed at 1000$\times$ g for 5 minutes. The liquid was washed with normal saline for three times until the supernatant became colorless. Counting was conducted by using a blood counting plate, the cell concentration was adjusted to $5 \times 10^6$ cells/ml, and tail intravenous injection was performed in 0.1 ml/kg so as to cause systemic fungal infection in the mice. The mice were randomly assigned to three groups: a posaconazole group, a test compound group and a solvent group, with 10 mice for each group. Since the posaconazole is insoluble in the solvent (normal saline), the posaconazole group in the experiment was a commercially available posaconazole injection, that was, sulfobutyl ether-β-cyclodextrin was used for solubilization. Two hours after the mouse systemic fungal infection model was constructed, each administration group was subjected to tail intravenous injection in 20 mg/kg (calculated as posaconazole), respectively. The dosing volume was 0.1 ml/kg, the model group was administered 0.9% sodium chloride solution in 0.1 ml/kg, once a day, and drug administration was performed for 5 days consectively. Death profile of the mice was observed, and survival duration was recorded. The observation was lasted for 7 days. Dead mice were all burned with ethanol.

3. Experimental results

Systemic fungal infection (intravenous administration): survival rate (%) of the mice in each group after administration

| Group | Dose (mg/kg) | Time (day) | | | | |
|---|---|---|---|---|---|---|
| | | 1 | 2 | 3 | 5 | 7 |
| Solvent group | - | 90% | 70% | 50% | 20% | 0% |
| Posaconazole | 20 | 100% | 100% | 100% | 100% | 90% |
| Example 7 | 20 | 100% | 100% | 100% | 100% | 90% |
| Example 8 | 20 | 100% | 100% | 100% | 100% | 90% |
| Example 9 | 20 | 100% | 100% | 100% | 100% | 90% |

It can be seen from the experimental data that the survival rate of the mice in the group for compound of the present invention is significantly higher than that in the solvent group. The survival rate of the mice in the compound

group on day 7 is the same as that in the posaconazole group. An excellent effect is achieved.

**Experiment 8:** Experiment of intragastric administration of the compound of the present invention to resist systemic fungal infection in mice

1. Experimental material

1.1 Experimental apparatuses

Multiskan MK3 ELISA detector, waterproof electro-heating standing-temperature cultivator, zo-F160 whole temperature shaking incubator, MJX intelligent mold incubator, SW-CT-IF ultraclean bench and ultraviolet spectrophotometer

1.2 Experiment reagent

Dimethyl sulfoxide and Sabouraud glucose solid agar culture medium (SDA)

1.3 Experimental animal

ICR mice, body weight of 18~22 g, female, provided by Jiangsu Experimental Animal Center

1.4 Experimental strain

Standard strain of candida albicans was purchased from American Type Culture Collection center, the strain number was ATCC10231.

2. Experimental method

Before the experiment, a small amount of candida albicans was picked by an inoculation loop from the SDA medium which was preserved at 4°C, the candida albicans was inoculated to 1 ml of YPD culture solution. The culture was vibrated at 200 rpm at 30°C. The fungi were activated for 16 hours and allowed to be enter a later phase of an exponential growth phase. The fungi were counted by using a blood cell counting plate, and the concentration of the bacteria liquid was adjusted to $1 \times 10^3$~$5 \times 10^3$CFU/ml with RPMI1640. The candida albicans monoclone on the SDA plate was picked and was cultured at 200 rpm at 35°C to reach the later phase of the exponential growth phase, and inoculated to 1 ml of YPD, 1% of the culture was inoculated to a fresh medium and cultured for 6 hours, and centrifugation was performed at 1000 × g for 5 minutes. The liquid was washed with normal saline for three times until the supernatant became colorless. Counting was conducted by using a blood counting plate, the cell concentration was adjusted to $5 \times 10^6$ cells/ml, and tail intravenous injection was performed in 0.1 ml/kg so as to cause systemic fungal infection in the mice.

The mice were randomly assigned to three groups: a posaconazole group, a test compound group and a solvent group, with 10 mice for each group. The posaconazole group in the experiment was a commercially available posaconazole injection, that was, sulfobutyl ether-β-cyclodextrin was used for solubilization, other test drug was dissolved in normal saline, untrasounded until it became clear and was ready for administration. Two hours after the mouse systemic fungal infection model was constructed, each administration group was subjected to intragastric administration in 20 mg/kg (calculated as posaconazole), respectively. The dosing volume was 0.1 ml/kg, the model group was administered 0.9% sodium chloride solution in 0.1 ml/kg, once a day, and drug administration was performed for 5 days consectively. Death profile of the mice was observed, and survival duration was recorded. The observation was lasted for 7 days. Dead mice were all burned with ethanol.

3. Experimental results

Systemic fungal infection (intragastric administration): survival rate (%) of mice in each group after administration

| Group | Dose (mg/Kg) | Time (day) | | | | |
|---|---|---|---|---|---|---|
| | | 1 | 2 | 3 | 5 | 7 |
| Solvent group | - | 90% | 70% | 50% | 20% | 0% |
| Posaconazole | 20 | 100% | 100% | 90% | 60% | 50% |

(continued)

| Group | Dose (mg/Kg) | Time (day) | | | | |
|---|---|---|---|---|---|---|
| | | 1 | 2 | 3 | 5 | 7 |
| Example 10 | 20 | 100% | 100% | 90% | 80% | 80% |

It can be seen from the experimental results that the survival rate of the mice in the group for the compound of the present invention is significantly higher than that of the solvent group. The survival rate of the mice in the compound group on Day 7 is higher than that in the posaconazole group, with higher bioavailability.

**Experiment 9:** In-vivo pharmacokinetics experiment

Test method: experimental animals were female mice with an age of 6~8 weeks and had a body weight of 190~215 g. The mice were purchased from Beijing Weili Tonghua Experimental Animal Technology Co., Ltd. Based on the weight, the mice were randomly divided into 5 groups, with each group including 3 animals. The dose and route of administration for the mice in each group is shown in the following table.

| Group | Drug | Drug dose (mg/Kg) | Route | Quantity |
|---|---|---|---|---|
| Group 1 | Compound in Example 1 | 1 | Intravenous | 3 |
| Group 2 | Comparative example 11 (compound E) | 1 | Intravenous | 3 |

Before the pharmacokinetic test, the mice were fasted for 16 hours. By referring to the above Table , compound of a single dose (1ml/kg; 1mg/kg) was intravenously administered. 200 μl of blood was collected at preset timepoint by jugular vein puncture after administration. For the animal group subjected to intravenous administration, blood was collected at minute 0, minute 15, minute 30, hour 1, hour 2, hour 4, hour 8 and hour 24 after administration. Urine was collected at hour 2, hour 4, hour 8, hour 12 and hour 24. The blood was collected in a sample tube containing EDTAand blood sample was immediately centrifuged at 4000 rpm at 4°C for 5 minutes. The plasma was transferred to another sample tube and stored at minus 20°C.

The concentration of posaconazole converted from the tested compound in the blood sample and in the urine sample obtained at each time point was detected, and the sample was subjected to pharmacokinetic test. The following method and apparatus were used:

HPLC: Shimadzu
MS: AB API4000Q
Column: Phenomenex Luna 5μm C18
Mobile phase: 100% of acetonitrile (3mM ammonium acetate) and 100% of water (3mM ammonium acetate)
Quantitation method: internal standard method
Results of the in-vivo pharmacokinetic test are as follows:

Test results for the compound E in Comparative example 11

| Time | Blood sample | | Time | Urine sample | |
|---|---|---|---|---|---|
| | Concentration of compound E | Concentration of posaconazole | | Concentration of compound E | Concentration of posaconazole |
| 0.25h | 5968.4 ng/ml | 0 ng/ml | 0~2h | 113059.5 ng/ml | 31290.0 ng/ml |
| 0.5h | 4868.3 ng/ml | 22.7 ng/ml | 2~4h | 91394.8 ng/ml | 1393.6 ng/ml |
| 1h | 22167.7 ng/ml | 104.3 ng/ml | 4~8h | 23826.5 ng/ml | 753.8 ng/ml |
| 2h | 70706.4 ng/ml | 127.5 ng/ml | 8~12h | 23806.2 ng/ml | 158.5 ng/ml |
| 4.0h | 24375.9 ng/ml | 169.1 ng/ml | 12~24h | 5237.1 ng/ml | 330.7 ng/ml |

(continued)

| Time | Blood sample | | Time | Urine sample | |
|---|---|---|---|---|---|
| | Concentration of compound E | Concentration of posaconazole | | Concentration of compound E | Concentration of posaconazole |
| 8.0h | 7350.1 ng/ml | 62.9 ng/ml | 24h morning urine | 0 | 46.4 ng/ml |
| 24h | 267.2 ng/ml | 15.7 ng/ml | | | |

Test results of the compound in Example 1 of the present invention

| Time | Blood sample | | Time | Urine sample | |
|---|---|---|---|---|---|
| | Concentration of the compound in Example 1 | Concentration of posaconazole | | Concentration of the compound in Example 1 | Concentration of posaconazole |
| 0.25h | 5564 ng/ml | O.Ong/ml | 0~2h | 149.1 ng/ml | 0.0 ng/ml |
| 0.5h | 7036 ng/ml | 35.18 ng/ml | 2~4h | 641.6 ng/ml | 0.0 ng/ml |
| 1h | 7632 ng/ml | 38.16 ng/ml | 4~8h | 973.0 ng/ml | 0.0 ng/ml |
| 2h | 8272 ng/ml | 41.36 ng/ml | 8~12h | 716.5 ng/ml | 0.0 ng/ml |
| 4.0h | 7524 ng/ml | 37.62 ng/ml | 12~24h | 400.9 ng/ml | 0.0 ng/ml |
| 8.0h | 7776 ng/ml | 38.88 ng/ml | 24h morning urine | 0 ng/ml | 0.0 ng/ml |
| 24h | 7924 ng/ml | 39.62 ng/ml | | | |

It can be seen from data in the table that the compound E in the Comparative example is difficult to be hydrolyzed into an active ingredient by enzymes in the body, but it can be rapidly metabolized via a circulatory system and enriched in the urine in vivo, greatly reducing the bioavailability of the compounds. However, the compound of the present invention is not enriched in the urine, which facilitates its use in medicine.

## Claims

**1.** A compound (posaconazole phosphate monocholinate) of formula (I):

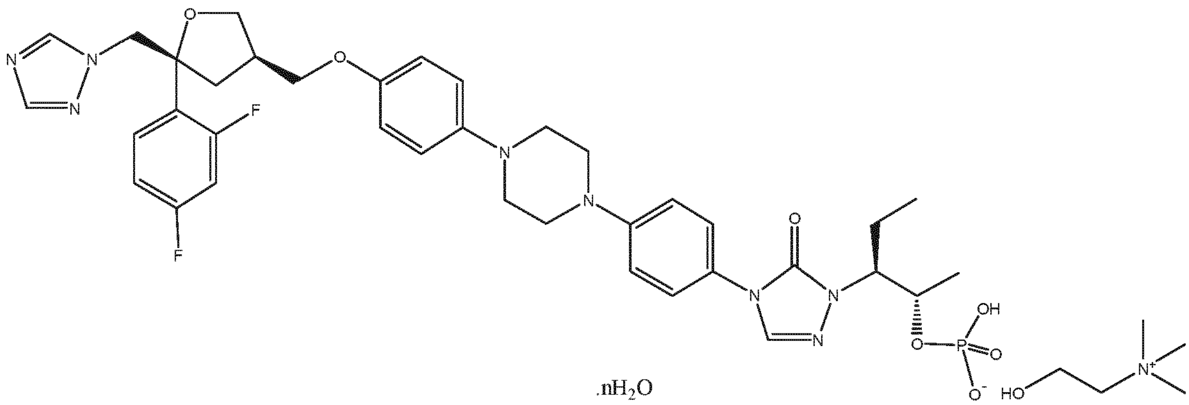

$.nH_2O$

(I)

wherein n is an integer of 0~12, preferably an integer of 0~8, more preferably an integer of 0~6.

**2.** A method for preparing the compound of formula (I) according to claim 1, comprising steps of:

(a) reacting a compound of formula A with a compound of formula B in the absence of a solvent or in an organic solvent A in the present of an inert gas so as to form a compound of formula C;

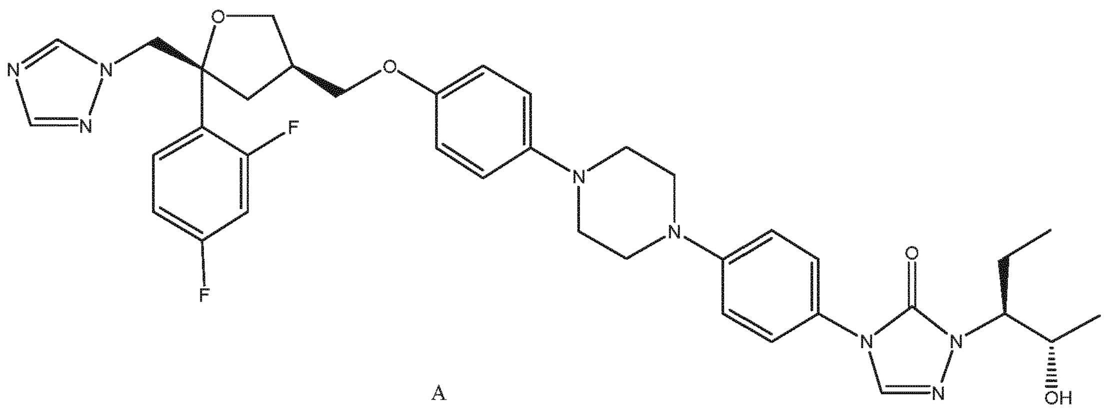

A

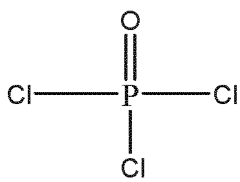

B

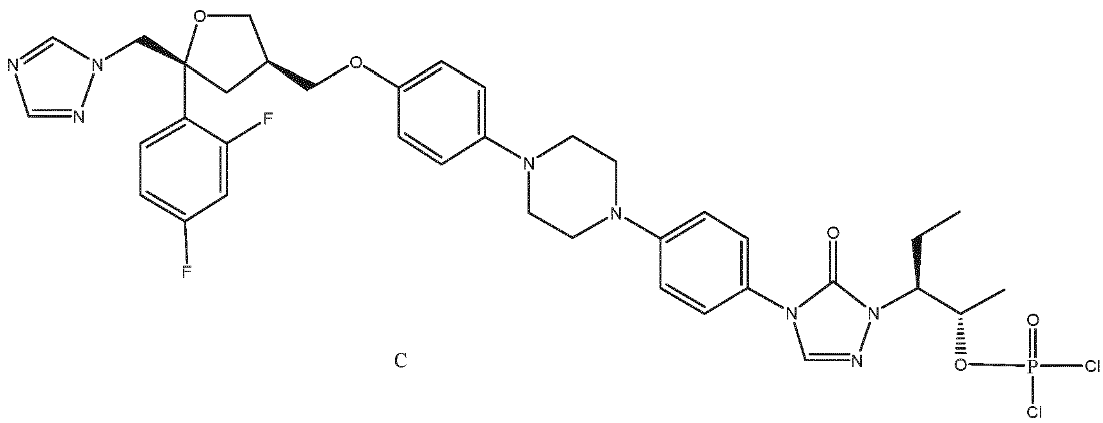

C

(b) hydrolyzing the compound of formula C formed in the step (a) by using a solvent B so as to form a compound of formula D:

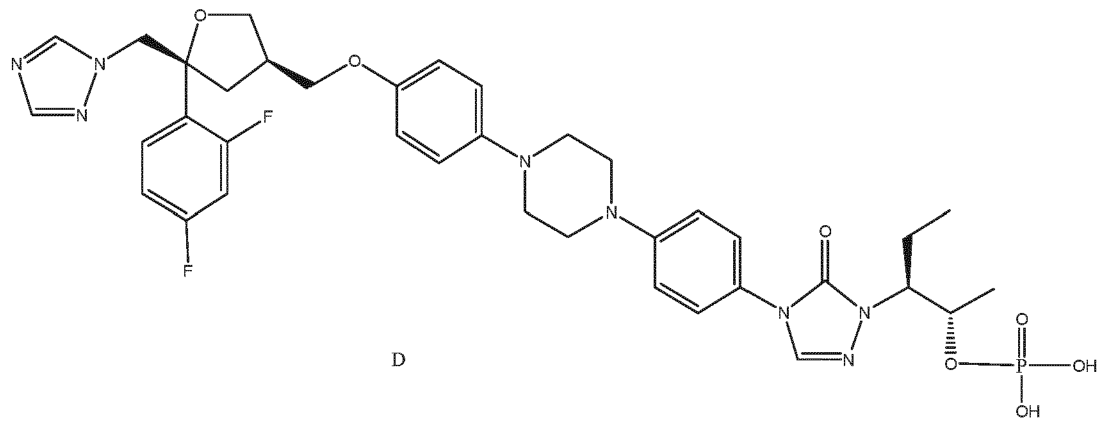

D

(c) reacting the compound of formula D obtained in the step (b) with choline hydroxide in a solvent C so as to prepare the compound of formula (I).

3. The method according to claim 2, wherein in the step (a), the inert gas is selected from one or more of nitrogen, helium or argon, preferably nitrogen or argon;

preferably, in the step (a), the organic solvent A is selected from one or more of an aromatic hydrocarbon solvent, a halohydrocarbon solvent, a nitrile solvent, a ketone solvent, an ether solvent, triethylamine, diethylamine, pyridine, 1-methylimidazole, N,N-diisopropyl ethylamine and an ester solvent, preferably ethyl acetate, acetonitrile, tetrahydrofuran, dichloromethane, toluene, acetone, triethylamine, 1-methylimidazole, pyridine or chloroform;
preferably, in the step (b), the solvent B is selected from one or more of water, an alkaline aqueous solution and an aqueous organic solvent solution; the alkaline aqueous solution is preferably an aqueous solution of sodium hydroxide, ammonium hydroxide, an aqueous solution of potassium hydroxide or an aqueous solution of sodium carbonate; and the aqueous organic solvent solution is preferably an aqueous solution of methanol, an aqueous solution of ethanol, an aqueous solution of isopropanol or an aqueous solution of acetone;
preferably, in the step (c), the solvent C is selected from one or more of water, aromatic hydrocarbons, halohydrocarbons, nitriles, ketones, ethers, alcohols and esters; preferably selected from one or more of water, acetonitrile, methanol, ethanol, acetone, isopropanol, tetrahydrofuran, ethyl acetate, dichloromethane, toluene and butanone.

4. The method according to claim 2 or 3, wherein in the step (a), reaction temperature is -10°C~50°C, preferably 0°C~35°C;
preferably, in the step (b), hydrolysis temperature is -20°C~30°C, preferably -5°C~10°C; preferably, in the step (c), reaction temperature is -10°C~80°C, preferably 10°C~40°C.

5. The method according to any of claims 2~4, wherein in the step (a), molar ratio of the compound of formula A to the compound of formula B is 1: (1.0~20.0), preferably 1:(2.25~10.0);
preferably, in the step (c), molar ratio of the compound of formula D to the choline hydroxide is 1:(0.5~2), preferably 1:1.05.

6. Use of the compound according to claim 1 in preparation of a drug resisting fungal infection; preferably, the fungal infection is an infection caused by *Candida* or *Cryptococcus.*

7. A pharmaceutical composition, comprising the compound of formula (I) according to claim 1 and a pharmaceutically acceptable excipient;
preferably, the pharmaceutical composition is a tablet, a suppository agent, a dispersible tablet, an enteric-coated tablet, a chewable tablet, an orally disintegrating tablet, a capsule, a sugar-coated tablet, a granule, dry powder, oral solution, a small volume injection, lyophilized powder for injection or a large volume parenteral solution; preferably, the pharmaceutically acceptable excipient is selected from one or more of the following excipients: a pH regulator, a diluent, a disintegrating agent, a suspending agent, a lubricating agent, a binder, a filler, a flavoring agent, a sweetening agent, an antioxidant, a preservative, a coating agent and coloring agent.

## Patentansprüche

1. Verbindung (Posaconazolphosphatmonocholinat) der Formel (I):

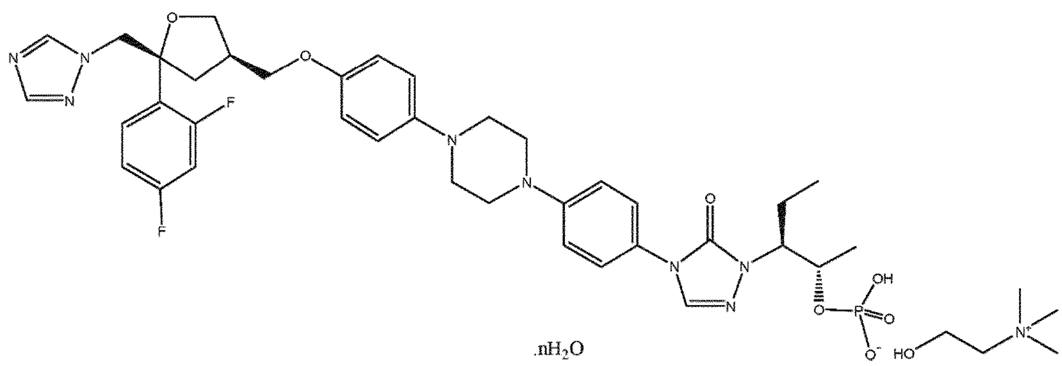

$.nH_2O$

(I)

wobei n für eine ganze Zahl von 0-12, bevorzugt eine ganze Zahl von 0-8, stärker bevorzugt eine ganze Zahl von 0-6 steht.

2. Verfahren zur Darstellung der Verbindung der Formel (I) nach Anspruch 1, umfassend die Schritte:

(a) Umsetzen einer Verbindung der Formel A mit einer Verbindung der Formel B in Abwesenheit eines Lösungsmittels oder in einem organischen Lösungsmittel A in Gegenwart eines Inertgases, so dass eine Verbindung der Formel C gebildet wird;

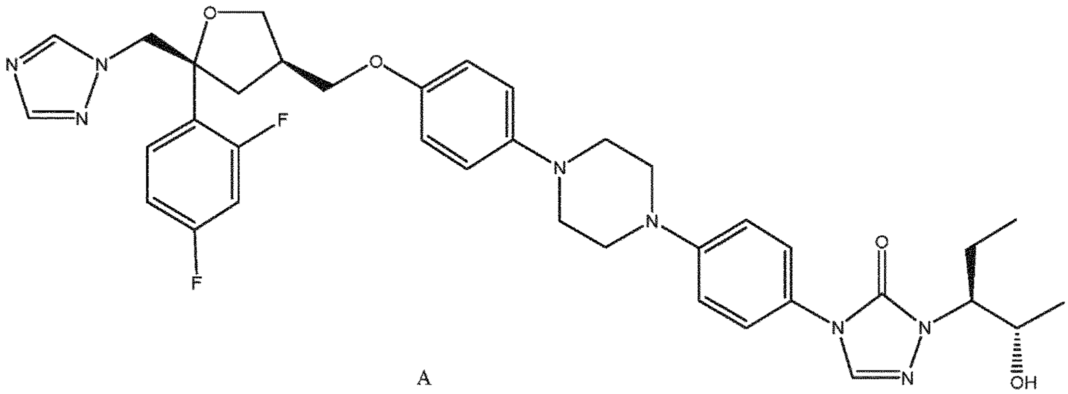

A

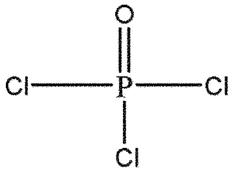

B

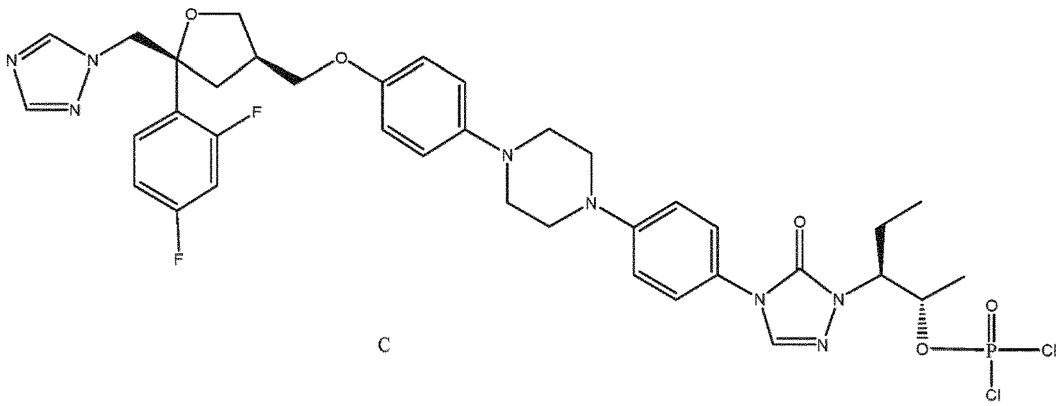

C

(b) Hydrolysieren der in Schritt (a) gebildeten Verbindung der Formel C unter Verwendung eines Lösungsmittels B, so dass eine Verbindung der Formel D gebildet wird:

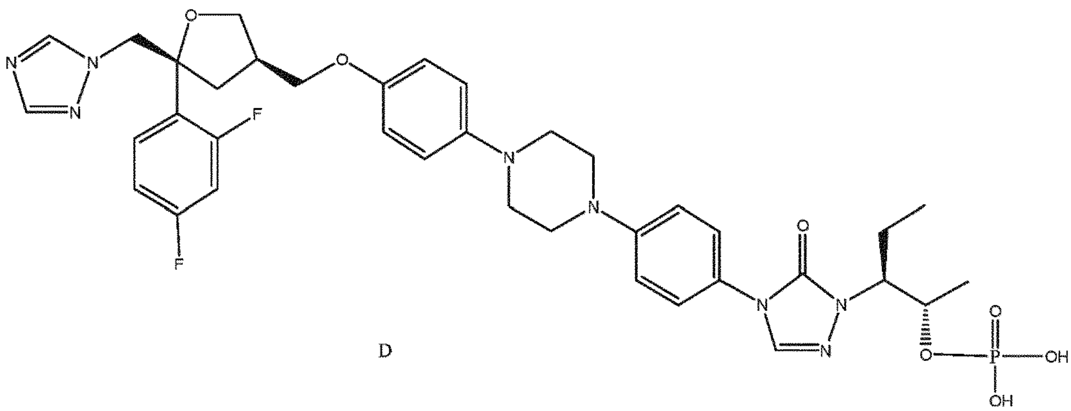

D

(c) Umsetzen der im Schritt (b) erhaltenen Verbindung der Formel D mit Cholinhydroxid in einem Lösungsmittel C, so dass die Verbindung der Formel (I) dargestellt wird.

**3.** Verfahren nach Anspruch 2, wobei im Schritt (a) das Inertgas aus einem oder mehreren von Stickstoff, Helium oder

Argon, bevorzugt Stickstoff oder Argon ausgewählt ist;

vorzugsweise im Schritt (a) das organische Lösungsmittel A aus einem oder mehreren von einem aromatischen Kohlenwasserstofflösungsmittel, einem Halogenkohlenwasserstofflösungsmittel, einem Ketonlösungsmittel, einem Etherlösungsmittel, Triethylamin, Diethylamin, Pyridin, 1-Methylimidazol, N,N-Diisopropylethylamin und einem Esterlösungsmittel, bevorzugt Essigester, Acetonitril, Tetrahydrofuran, Dichloromethan, Toluol, Aceton, Triethylamin, 1-Methylimidazol, Pyridin oder Chloroform ausgewählt ist;

vorzugsweise im Schritt (b) das Lösungsmittel B aus einem oder mehreren von Wasser, einer alkalischen wässrigen Lösung und einer wässrigen Lösung eines organischen Lösungsmittels ausgewählt ist; es sich bei der alkalischen wässrigen Lösung bevorzugt um eine wässrige Lösung von Natriumhydroxid, Ammoniumhydroxid, eine wässrige Lösung von Kaliumhydroxid oder eine wässrige Lösung von Natriumcarbonat handelt; und es sich bei der wässrigen Lösung eines organischen Lösungsmittels bevorzugt um eine wässrige Lösung von Methanol, eine wässrige Lösung von Ethanol, eine wässrige Lösung von Isopropanol oder eine wässrige Lösung von Aceton handelt;

vorzugsweise im Schritt (c) das Lösungsmittel C aus einem oder mehreren von Wasser, aromatischen Kohlenwasserstoffen, Halogenkohlenwasserstoffen, Nitrilen, Ketonen, Ethern, Alkoholen und Estern; bevorzugt aus einem oder mehreren von Wasser, Acetonitril, Methanol, Ethanol, Aceton, Isopropanol, Tetrahydrofuran, Essigsester, Dichlormethan, Toluol und Butanon ausgewählt ist.

**4.** Verfahren nach Anspruch 2 oder 3, wobei im Schritt (a) die Reaktionstemperatur bei -10°C-50°C, bevorzugt 0°C-35°C liegt;

vorzugsweise im Schritt (b) die Hydrolysetemperatur bei -20°C-30°C, bevorzugt - 5°C-10°C liegt;
vorzugsweise im Schritt (c) die Reaktionstemperatur bei -10°C-80°C, bevorzugt 10°C-40°C liegt.

**5.** Verfahren nach einem der Ansprüche 2-4, wobei im Schritt (a) das Molverhältnis der Verbindung der Formel A zur Verbindung der Formel B bei 1:(1,0-20,0), bevorzugt 1:(2,25-10,0) liegt;
vorzugsweise im Schritt (c) das Molverhältnis der Verbindung der Formel D zum Cholinhydroxid bei 1:(0,5-2), bevorzugt 1:1,05 liegt.

**6.** Verwendung der Verbindung nach Anspruch 1 bei der Herstellung eines einer Pilzinfektion widerstehenden Arzneistoffs; vorzugsweise handelt es sich bei der Pilzinfektion um eine durch *Candida* oder *Cryptococcus* verursachte Infektion.

**7.** Pharmazeutische Zusammensetzung, umfassend die Verbindung der Formel (I) nach Anspruch 1 und einen pharmazeutisch unbedenklichen Exzipienten;
vorzugsweise handelt es sich bei der pharmazeutischen Zusammensetzung um eine Tablette, ein Zäpfchen, eine dispergierbare Tablette, eine magensaftresistente Tablette, eine Kautablette, eine sich im Mund auflösende Tablette, eine Kapsel, ein Dragee, ein Granulat, Trockenpulver, orale Lösung, eine kleinvolumige Injektion, lyophilisiertes Pulver zur Injektion oder eine großvolumige parenterale Lösung; vorzugsweise ist der pharmazeutisch unbedenkliche Exzipient aus einem oder mehreren der folgenden Exzipienten ausgewählt: einem pH-Regulator, einem Verdünnungsmittel, einem Sprengmittel, einem Suspensionsmittel, einem Gleitmittel, einem Bindemittel, einem Füllstoff, einem Geschmacksstoff, einem Süßstoff, einem Antioxidans, einem Konservierungsstoff, einem Überzug und Farbstoff.

## Revendications

**1.** Composé (monocholinate de phosphate de posaconazole) de formule (I):

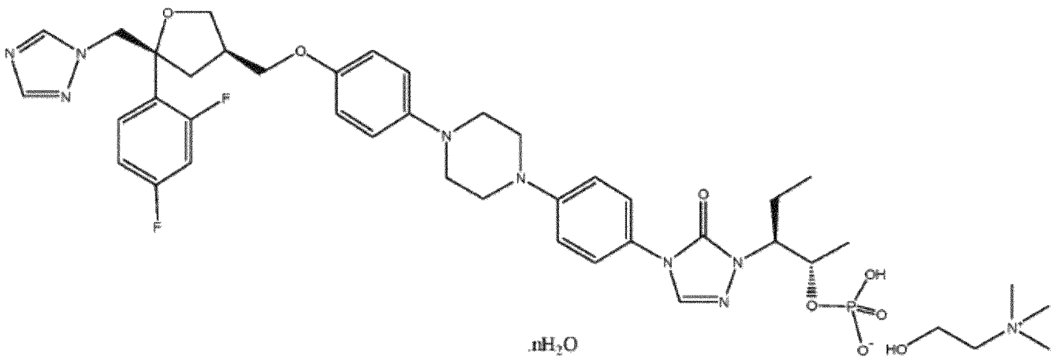

(I)

n étant un entier de 0 à 12, préférablement un entier de 0 à 8, plus préférablement un entier de 0 à 6.

**2.** Procédé pour la préparation du composé de formule (I) selon la revendication 1, comprenant les étapes de :

(a) mise en réaction d'un composé de formule A avec un composé de formule B en l'absence d'un solvant ou dans un solvant organique A en la présence d'un gaz inerte de sorte à former un composé de formule C :

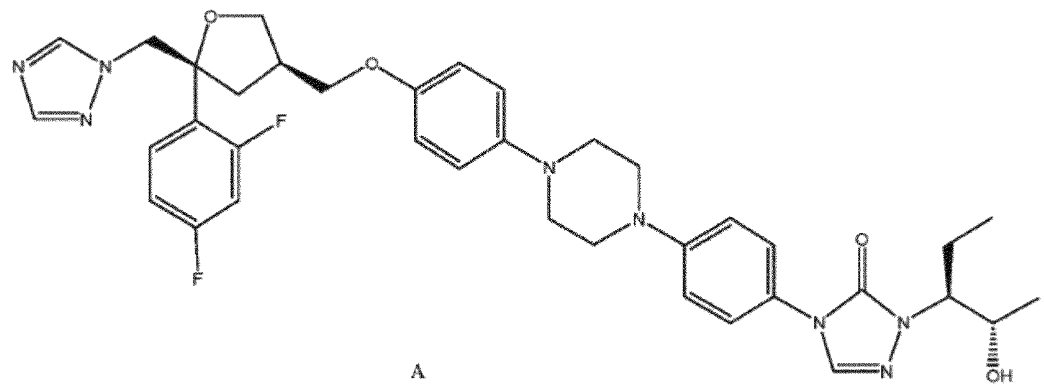

A

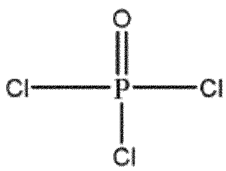

B

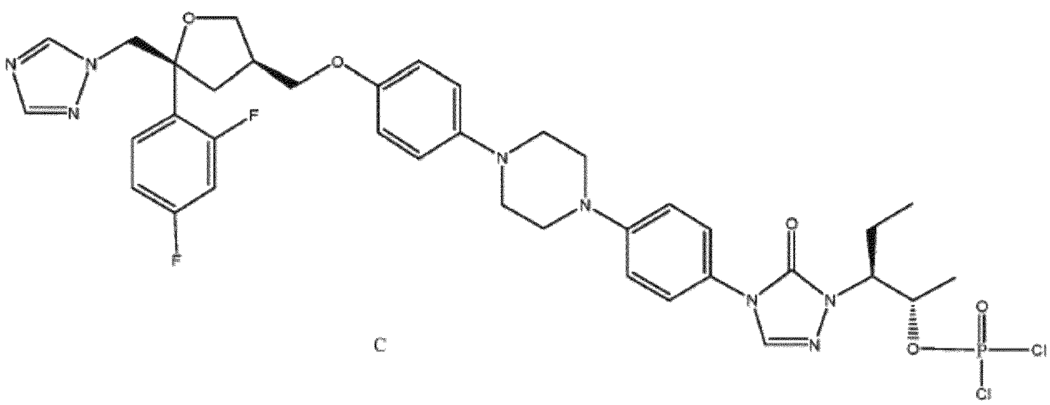

C

(b) hydrolyse du composé de formule C formé dans l'étape (a) en utilisant un solvant B de sorte à former un composé de formule D :

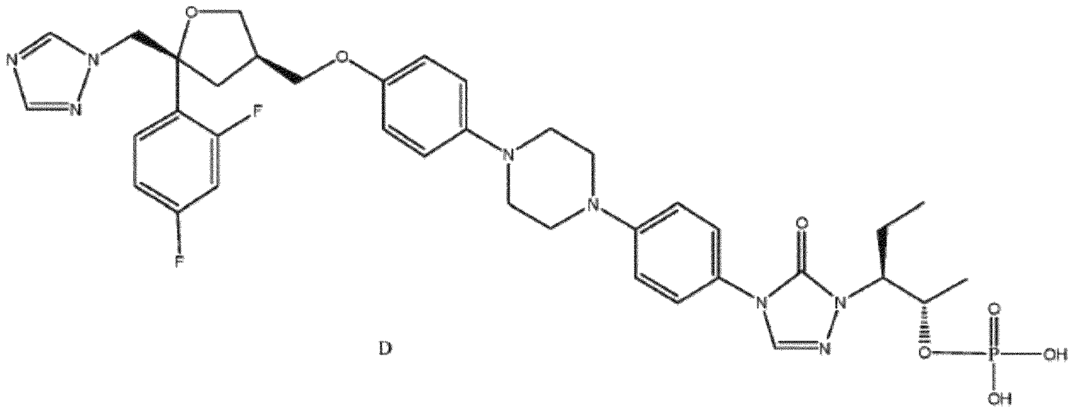

D

(c) mise en réaction du composé de formule D obtenu dans l'étape (b) avec de l'hydroxyde de choline dans un solvant C de sorte à préparer le composé de formule (I).

**3.** Procédé selon la revendication 2, dans lequel dans l'étape (a) le gaz inerte est choisi parmi un ou plusieurs parmi l'azote, l'hélium et l'argon, préférablement l'azote ou l'argon ;

préférablement, dans l'étape (a), le solvant organique A est choisi parmi l'un ou plusieurs parmi un solvant hydrocarboné aromatique, un solvant halogénohydrocarboné, un solvant de type nitrile, un solvant de type cétone, un solvant de type éther, la triéthylamine, la diéthylamine, la pyridine, le 1-méthylimidazole, la N,N-diisopropyléthylamine et un solvant de type ester, préférablement l'acétate d'éthyle, l'acétonitrile, le tétrahydrofuranne, le dichlorométhane, le toluène, l'acétone, la triéthylamine, le 1-méthylimidazole, la pyridine ou le chloroforme ;
préférablement, dans l'étape (b), le solvant B est choisi parmi l'un ou plusieurs parmi l'eau, une solution aqueuse alcaline et une solution aqueuse de solvant organique ; la solution aqueuse alcaline étant préférablement une solution aqueuse d'hydroxyde de sodium, l'hydroxyde d'ammonium, une solution aqueuse d'hydroxyde de potassium ou une solution aqueuse de carbonate de sodium ; et la solution aqueuse de solvant organique étant préférablement une solution aqueuse de méthanol, une solution aqueuse d'éthanol, une solution aqueuse d'isopropanol ou une solution aqueuse d'acétone ;
préférablement, dans l'étape (c), le solvant C est choisi parmi l'un ou plusieurs parmi l'eau, des hydrocarbures aromatiques, des halogénohydrocarbures, des nitriles, des cétones, des éthers, des alcools et des esters ; préférablement choisi parmi l'un ou plusieurs parmi l'eau, l'acétonitrile, le méthanol, l'éthanol, l'acétone, l'isopropanol, le tétrahydrofuranne, l'acétate d'éthyle, le dichlorométhane, le toluène et la butanone.

**4.** Procédé selon la revendication 2 ou 3, dans lequel dans l'étape (a), la température de réaction est de -10 °C à 50 °C, préférablement de 0 °C à 35 °C ;

préférablement, dans l'étape (b), la température d'hydrolyse est de -20 °C à 30 °C, préférablement de -5 °C à 10 °C ;
préférablement, dans l'étape (c), la température de réaction est de -10 °C à 80 °C, préférablement de 10 °C à 40 °C.

**5.** Procédé selon l'une quelconque des revendications 2 à 4, dans lequel dans l'étape (a), un rapport molaire du composé de formule A sur le composé de formule B est de 1 : (1,0 à 20,0), préférablement de 1 : (2,25 à 10,0) ; préférablement, dans l'étape (c), un rapport molaire du composé de formule D sur l'hydroxyde de choline est de 1 : (0,5 à 2), préférablement de 1 : 1,05.

**6.** Utilisation du composé selon la revendication 1 dans la préparation d'une infection fongique résistante aux médicaments ; préférablement, l'infection fongique étant une infection causée par *Candida* ou *Cryptococcus*.

**7.** Composition pharmaceutique, comprenant le composé de formule (I) selon la revendication 1 et un excipient pharmaceutiquement acceptable;

préférablement, la composition pharmaceutique étant un comprimé, un agent suppositoire, un comprimé dispersible, un comprimé revêtu de manière entérique, un comprimé à mâcher, un comprimé se désintégrant oralement, une capsule, un comprimé revêtu de sucre, un granule, une poudre sèche, une solution orale, une injection de petit volume, une poudre lyophilisée pour une injection ou une solution parentérale de grand volume ; préférablement, l'excipient pharmaceutiquement acceptable étant choisi parmi l'un ou plusieurs parmi les excipients suivants : un régulateur de pH, un diluant, un agent désintégrant, un agent de mise en suspension, un agent lubrifiant, un liant, une charge, un agent aromatisant, un agent édulcorant, un antioxydant, un conservateur, un agent de revêtement et un agent colorant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201180031488
- CN 106432338 A